(19) Europäisches Patentamt
European Patent Office
Office européen des brevets

(11) Veröffentlichungsnummer: **0 094 348**
**B1**

# (12) EUROPÄISCHE PATENTSCHRIFT

(45) Veröffentlichungstag der Patentschrift: **24.07.85**

(51) Int. Cl.⁴: **C 07 C 131/00**, C 07 C 135/00, A 01 N 35/10, C 07 D 295/08

(21) Anmeldenummer: **83810185.5**

(22) Anmeldetag: **02.05.83**

## (54) Neue Oximäther, Verfahren zu ihrer Herstellungs, Mittel die die neuen Oximäther enthalten und ihre Verwendung.

(30) Priorität: **06.05.82 CH 2804/82**

(43) Veröffentlichungstag der Anmeldung: **16.11.83 Patentblatt 83/46**

(45) Bekanntmachung des Hinweises auf die Patenterteilung: **24.07.85 Patentblatt 85/30**

(84) Benannte Vertragsstaaten: **BE CH DE FR GB IT LI NL**

(56) Entgegenhaltungen:
**EP - A - 0 015 456**
**US - A - 3 748 361**

(73) Patentinhaber: **CIBA-GEIGY AG, Postfach, CH-4002 Basel (CH)**

(72) Erfinder: **Martin, Henry, Dr., Jupiterstrasse 17, CH-4123 Allschwil (CH)**
Erfinder: **Fricker, Urs, Baumgartenweg 8, CH-4460 Gelterkinden (CH)**

EP 0 094 348 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft Oximäther, Verfahren zu ihrer Herstellung, Mittel zum Schutz von Kulturpflanzen vor der phytotoxischen Wirkung von Herbiziden, die diesen Oximäther als aktive Komponente enthalten und ihre Verwendung.

Es ist bekannt, dass Herbizide aus den verschiedensten Stoffklassen, wie Triazine, Harnstoffderivate, Carbamate, Thiolcarbamate, Halogenacetanilide, Halogenphenoxyessigsäuren usw. bei der Anwendung in wirksamer Dosis gelegentlich neben den zu bekämpfenden Unkräutern auch die Kulturpflanzen in gewissem Masse schädigen. Überdosen werden oft ungewollt und zufälligerweise appliziert, wenn sich Randzonen beim streifenweisen Spritzen überdecken, sei es durch Windeinwirkung oder durch falsches Einschätzen der Breitenwirkung des Spritzgerätes. Es können klimatische Verhältnisse oder eine Bodenbeschaffenheit vorliegen, so dass die für normale Bedingungen empfohlene Herbizidmenge als Überdosis wirkt. Qualität des Saatgutes kann bei der Herbizidverträglichkeit auch eine Rolle spielen. Um diesem Problem zu begegen, sind schon verschiedene Stoffe vorgeschlagen worden, welche befähigt sind, die schädigende Wirkung des Herbizids auf die Kulturpflanze spezifisch zu antagonisieren, d.h. die Kulturpflanze zu schützen, ohne dabei die Herbizidwirkung auf die zu bekämpfenden Unkräuter merklich zu beeinflussen. Dabei hat es sich gezeigt, dass die vorgeschlagenen Gegenmittel sowohl bezüglich der Kulturpflanzen als auch bezüglich des Herbizids und ggf. auch in Abhängigkeit von der Applikationsart oft sehr artspezifisch wirken, d.h. ein bestimmtes Gegenmittel eignet sich oft nur für eine bestimmte Kulturpflanze und einige wenige herbizide Stoffklassen.

So beschreibt die Britische Patentschrift Nr. 1277557 die Behandlung von Samen bzw. Sprösslingen von Weizen und Sorghum mit gewissen Oxamsäureestern und Amiden zum Schutz vor dem Angriff durch „Alachlor" (N-Methoxymethyl-N-chloracetyl-2,6-diäthylanilin). In den Deutschen Offenlegungsschriften Nrn. 1952910 und 2245471 sowie in der Französischen Patentschrift Nr. 2021611 werden Gegenmittel zu Behandlung von Getreide-, Mais- und Reissamen zum Schutz gegen die schädigende Einwirkung von herbizid wirksamen Thiolcarbamaten vorgeschlagen. Gemäss der Deutschen Patentschrift Nr. 1576676 und der US-Patentschrift Nr. 3131509 werden Hydroxyaminoacetanilide und Hydantoine für den Schutz von Getreidesamen gegenüber Carbamaten verwendet.

Die direkte pre- oder postemergente Behandlung gewisser Nutzpflanzen mit Gegenmitteln als Antagonisten bestimmter Herbizidklassen auf einer Anbaufläche ist in den Deutschen Offenlegungsschriften Nrn. 2141586 und 2218097 sowie im US-Patent Nr. 3867444 beschrieben.

Ferner können Maispflanzen gemäss der Deutschen Offenlegungschrift Nr. 2402983 wirksam vor Schädigung durch Chloracetanilide geschützt werden, indem man dem Boden als Gegenmittel ein N-disubstituiertes Dichloracetamid zuführt.

Gemäss DE-OS Nrn. 2808317 und 2837204 können auch Alkoximinobenzylcyanide, deren Alkoxygruppe verschiedentlich substituiert ist, als Mittel zum Schutz von Kulturpflanzen vor der schädigenden Wirkung von Herbiziden verschiedener Stoffklassen verwendet werden.

Gemäss vorliegender Erfindung werden Oximäther der Formel I

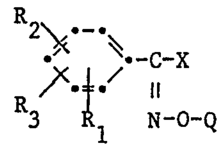

(I)

in welcher $R_1$ Wasserstoff, Halogen, niederes Alkyl, niederes Halogenalkyl, niederes Alkoxy, niederes Halogenalkoxy, niederes Alkylthio, niederes Halogenalkylthio, niederes Alkylsulfinyl, niederes Alkylsulfonyl, niederes Halogenalkylsulfinyl, niederes Halogenalkylsulfonyl oder Nitro,

$R_2$ und $R_3$ je Wasserstoff, Halogen, niederes Alkyl, niederes Alkoxy oder niederes Halogenalkyl,

Q ein niederes Alkyl, das geradkettig oder verzweigt ist oder durch Heteroatome unterbrochen und

a) durch Halogenatome, die Cyan- oder Oxygruppe substituiert ist oder

b) durch eine Phenoxy- oder Thiophenoxygruppe substituiert ist oder ferner

– ein niederes Alkenyl oder Halogenalkenyl,
– ein niederes Alkinyl oder Halogenalkinyl,
– einen 3-7gliedrigen Cycloalkylrest,
– einen niederen Alkancarbonsäureesterrest
– einen niederen Alkenylcarbonsäureesterrest,
– einen niederen Alkancarbonsäureamid- oder Alkancarbonsäurehydrazidrest,
– einen niederen Alkancarbonsäurethioamidrest,
– einen niederen Alkancarbonsäurethiolesterrest,
– einen niederen Alkancarbonsäurerest oder ein Salz davon,
– einen aliphatischen oder araliphatischen Acylrest,
– einen halogensubstituierten aliphatischen Acylrest,
– einen durch Alkoxy oder Phenoxy substituierten aliphatischen Acylrest,
– einen cycloaliphatischen, aromatischen oder heterocyclischen Acylrest,
– einen substituierten oder unsubstituierten Arylrest,
– einen aliphatischen, cycloaliphatischen oder aromatischen Kohlensäurerest,
– einen aliphatischen, cycloaliphatischen oder aromatischen Thiokohlensäurerest,
– den Carbamoylrest, einen Cyanoalkylcarbamoylrest oder falls X nicht die Trifluormethylgruppe darstellt, auch einen Alkylcarbamoylrest,
– einen Alkyl- oder Arylsulfonylrest,
– einen Alkoxyalkylsulfonylamido oder einen Sulfonylamidorest,
– einen halogensubstituierten Alkyl- oder Arylsulfonylrest,

– einen cyclischen Acylamidomethylrest

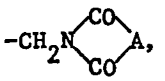

worin

A eine Alkylenkette, eine halogenierte Alkylenkette, eine Alkenylkette oder eine halogenierte Alkenylenkette bedeutet,

– einen Phthalimidomethylrest der teilweise oder ganz gesättigt sein kann und im Phenylkern gemäss $R_1$, $R_2$ und $R_3$ substituiert sein kann,

– einen 5-6gliedrigen gesättigten, teilweise gesättigten oder aromatischen Heterocyclus, der 1-3 Stickstoff, Sauerstoff und/oder Schwefelatome enthalten kann und der gemäss $R_1$, $R_2$ und $R_3$ substituiert sein kann, und

X einen fluorierten $C_1$-$C_3$-Alkylrest, der auch Chlor enthalten kann, bedeutet, vorgeschlagen.

Unter Halogen sind Fluor, Chlor, Brom und Jod, insbesondere aber Fluor und Chlor zu verstehen.

Der fluorierte Alkylrest X kann beispielsweise Difluormethyl, Trifluormethyl, Chlordifluormethyl, Tetrafluoäthyl, Pentafluoräthyl, Difluorchlorfluoräthyl und Heptafluorpropyl bedeuten. Unter den genannten Resten X sind perfluorierte Alkylreste bevorzugt, wobei ein Fluoratom durch Chloratom ersetzt sein kann. Besonders bevorzugte Reste X sind Trifluormethyl, Chlordifluormethyl, Pentafluoräthyl und Difluormethyl.

Der Ausdruck Alkyl allein oder als Teil eines Substituenten umfasst verzweigte oder unverzweigte $C_1$- bis $C_8$-Alkylgruppen, Niederalkyl bedeutet $C_1$-$C_4$-Alkyl. Beispiele sind Methyl, Äthyl, Propyl, Isopropyl, Butyl, Isobutyl, sec.-Butyl, tert.-Butyl sowie die höheren Homologen Amyl, Isoamyl, Hexyl, Heptyl, Octyl samt ihren Isomeren. Sinngemäss enthalten Alkanoyle oder Cyanalkyle ein zusätzliches C-Atom. Ein niederer Alkancarbonsäureester besteht aus einem Niederalkylteil mit 1-4 C-Atomen, der Carbonylgruppe und einem alkoholischen oder phenolischen Rest mit 1 bis 8 C-Atomen. Zu nennen wären besonders Essigester $-CH_2-COO-C_1$-$C_8$-Alkyl,
$-CH_2-CO-C_3-C_4$-Alkenyl,
$-CH_2-COO-C_3$-$C_4$-Alkinyl oder
$-CH_2-OO$-Phenyl,
wobei der Phenylrest durch $R_1$, $R_2$ und $R_3$ substituiert sein kann, ferner
$CH_2-COS-C_1$-$C_8$-Alkyl,
$-CH_2-COS-C_3$-$C_4$-Alkenyl, oder
$-CH_2-COSC_3$-$C_4$-Alkinyl,
ebenso die entsprechenden 1-Propionsäureester $-CH(CH_3)-COT$ (T = einen $C_1$-$C_8$-Alkyloxy-, $C_1$-$C_8$-Alkylthio-, $C_3$-$C_4$-Alkenyloxy-, $C_3$-$C_4$-Alkenylthio-, $C_3$-$C_4$-Alkinyloxy-, $C_3$-$C_4$-Alkinylthio- oder einen ggf. durch $R_1$, $R_2$ und $R_3$ substituierten Phenoxyrest). Alkenyl bedeuten aliphatische Reste mit ein oder auch zwei Doppelbindungen (Alkadienyle) und maximal 6, bevorzugt 4 C-Atomen, Halogenalkenyle enthalten bis zu 3 Halogenatome, bevorzugt Chlor oder Brom. Niederes Alkinyl bedeutet Propinyl (= Propargyl) und Butinyl.

Ein niederer Alkenylcarbonsäureester besteht demnach aus einem Niederalkenylteil mit 3-4 C-Atomen der Carbonylgruppe und einem alkoholischen oder phenolischen Rest mit 1-8 C-Atomen. Zu nennen sind besonders die Butenoyl- und Pentenoylesterreste
$-CH_2-CH=CH-COT$,
$CH_2-CH(CH_3)=CH-COT$,
$CH(CH_3)=CH=CH-COT$,
worin T die oben gegebene Bedeutung hat. Ein niederer Alkancarbonsäurerest oder ein Salz davon besteht aus dem Niederalkylteil mit 1-4 C-Atomen und der Carboxylgruppe oder einem Salz davon mit einem Alkalimetall-, Erdalkalimetall-, Eisen oder Kupfer, Ion der dem Kation einer quaternären Ammoniumgruppe.

Alkancarbonsäureamide umfassen auch mono- oder symmetrisch oder unsymmetrisch di-substituierte Amide, wobei die Substituenten wahlweise Niederalkyl, Niederalkenyl, Propinyl oder Butinyl sowie einmal einen Cycloalkyl- oder Phenylring sein können, der gemäss Definition für $R_2$, $R_3$ substituiert oder unsubstituiert sein kann. Als Beispiele dafür sind zu verstehen der Essigsäureamidrest $-CH_2CONH_2$, der Essigsäurethiamidrest $-CH_2CSNH_2$, oder die entsprechenden Reste, in welchen ein oder beide Reste durch Niederalkyl, Niederalkenyl, Niederalkinyl, Cycloalkyl oder Phenyl ersetzt sind, ferner die entsprechenden 1-Propionsäureamidreste $-CH(CH_3)CONH_2$ und 1-Propionsäurethioamidreste $-CH(CH_3)-CS-NH_2$. Unter diesen Begriff fallen ebenfalls die acylierten Alkancarbonsäureamide wie beispielsweise der Essigsäureacetamidrest $-CH_2-CONHCOCH_3$, der 1-Propionylacetamidrest $-CH(CH_3)CONHCOCH_3$, der Essigsäureureidorest $-CH_2CONHCO-NH_2$, der 1-Propionylureidorest $-CH(CH_3)$-$CONHCONH_2$, welche symmetrisch oder asymmetrisch an den Stickstoffatom durch Niederalkyl, Niederalkenyl, Niederalkinyl, Cycloalkyl oder Phenyl substituiert sein können, ferner der Essigsäure- oder 1-Propionsäureformamidorest sowie der Essigsäure- oder 1-Propionsäurehydrazidorest.

Ein aliphatischer, araliphatischer, cycloaliphatirestscher oder aromatischer Acylrest besteht aus einer Carbonylgruppe welche mit einer Alkylgruppe mit bis 8 C-Atomen, einer halogenierten Alkylgruppe, einer Alkoxygruppe, einer halogenierten Alkoxygruppe, einer Alkenylgruppe mit 3-6 C-Atomen, einer halogenierten Alkenylgruppe, einer Alkenyloxygruppe, einer halogenierten Alkenyloxygruppe, einer Alkinylgruppe mit 3-6 C-Atomen, einer halogenierten Alkinylgruppe, einer Alkinyloxygruppe, einer halogenierten Alkinyloxygruppe, einer Cycloalkylgruppe mit 3 bis 8 C-Atomen, einer halogenierten Cyclialkylgruppe mit 3 bis 8 C-Atomen, einer halogenierten Cycloalkylgruppe oder einer ggf. durch $R_1$, $R_2$ und $R_3$ substituierten Phenylgruppe, verbunden ist. Als Beispiele für solche Reste sind zu nennen Acetyl, Propionyl, Neopentamoyl, 1,1-Dichlorpropionyl, Crotonyl, 2-Methylcrotonyl, Chloracetyl, 1,1-Dimethyl-2-chlorpropionyl, Dichloracetyl, Trifluoracetyl, Acroyl, Methacroyl, Dimethylacroyl, 1,2,2-Trichloracroyl, Dibromacroyl, α-Bromacroyl, Methyloxylyl, Äthyloxolyl, Amidooxalyl, Methoxyacetyl, Phenoxyacetyl, 1-(Phenoxy)propionyl, 1-(4-Chlorphenoxy)propionyl, 1-(2-Chlorphenoxy)propionyl, 1-(2,4-Dichlorphenoxy)propionyl, 1-(4-Chlor-2-methylphenoxy)propionyl, Phenylthioacetyl, 1-(4-Chlorphenylthio)acetyl, Phenylacetyl, 4-Chlorphenylacetyl, 3-Methyl-1-phenylbutyryl, 3-Methyl-1-parachlorphenylbutyryl, 2,2-Dimethylcyclopropylcarbonyl, Cyclopropylcarbonyl, 2,2-Dichlorcyclopropylcarbonyl, 1-Methylcyclopropylcabonyl, Cyclopentylcarbonyl, Cyclohexylcarbonyl, Benzoyl, 2-Methylbenzoyl, 3-Methylbenzoyl, 3-Trifluormethylbenzoyl, 2-Trifluormethylbenzoyl, 4-Difluorchlormethylbenzoyl, 2-Nitrobenzoyl, 4-Chlorbenzoyl, 3-Chlorbenzoyl, 4-Amisoyl, 3-Acetoxybenzoyl, 3-Methyl-4-nitrobenzoyl.

Ein heterocyclischer Acylrest besteht aus der Carbonylgruppe, die mit einem heterocyclischen Rest verbunden ist. Darunter werden gesättigte, teilgesättigte oder heteroaromatische 5- bis 6-gliedrige Reste verstanden, die 1 bis 3 Stickstoff, Sauerstoff oder Schwefelatom enthalten und welche durch Halogenatom, niederes Alkyl oder wie unter $R_1$, $R_2$ und $R_3$ angegeben substituiert sein können und die auch Oxo- oder Sulfoxogruppen enthalten können.

Beispiele für solche Reste sind Nicotinoyl, Isonicotinoyl, 1,3-Dichlo-4-pyridinecarbonyl; Thiophen-2-carbonyl; 2-Furoyl, 4-Methyl-1,2,3-thiadiazol-5-carbonyl, 4-(2,5-Dimethyl)furoryl, 4-(3,5-Dimethyloxazol)carbonyl, 3-(2-Methyl-2,3 dihydro-4-[H]-pyran)carbonyl, 2-(3-Methyl-3,4-dihydro-1,4-thioxan)carbonyl, 2-(3-Methyl-3,4-dihydro-1-oxo-1,4-thioxan)carbonyl, 2-(2,4-Dimethylthiazol)carbonyl, 2-Thiazolcarbonyl, 4-(2,4-Dichlorpyrimidin)carbonyl, 2-(3-Brom)furoyl, 2-(4-Nitro)furoyl, 2-(3-Methyl)furoyl, 2-(3-Chlor)thiophencarbonyl, 4-Pyridazincarbonyl, 4-Piperidincarbonyl, 2-(4-Methyl)piperidincarbonyl, 2-Morpholincarbonyl, 2-Thiomorpholincarbonyl, 2-Pyrrolcarbonyl, 2-Tetrahydrofuroyl, 2-Oxazoyl, 3-Pyrazolylcarbonyl, 2-Pyrrolcarbonyl, 2-Pyrrolidincarbonyl.

$C_3$-$C_7$-Cycloalkylgruppen sind Cyclopropyl, Cyclobutyl, Cyclopentyl, Cyclohexyl und Cycloheptyl. Cycloaliphatische Reste entsprechen diesen Ringsystemen, können daneben aber noch, je nach Möglichkeit, eine oder mehrere Doppelbindungen enthalten.

Ein araliphatischer Rest umfasst eine Arylgruppe wie ggf. gemäss $R_1$, $R_2$ und $R_3$ ein- bis dreifach substituiertes Phenyl, oder auch Naphthyl, Fluorenyl, Indanyl, das über Niederalkyl oder Niederalkenyl an den Rest des Moleküls gebunden ist. Beispiele sind die Grundkörper Benzyl, Phenäthyl, Phenylallyl ferner 4-Chlorbenzyl, 3,4-Chlorbenzyl, 3-Trifluorbenzyl, 2-Chlorbenzyl, 3-Chlorbenzyl, 3-Brombenzyl, 4-Cyanobenzyl, 4-Methylbenzyl, 4-tert.-Butylbenzyl, 2-Fluorbenzyl, 2-Fluorphenyläthyl, 2-Nitrobenzyl, 4-Methoxybenzyl, 3-Methoxybenzyl, 4-Phenylbenzyl, 4-Fluorbenzyl.

Beispiele für durch Heteroatome unterbrochene aliphatische Ketten seien Methoxyäthyl, Äthoxyäthyl, Propoxyäthyl, Butoxyäthyl, Methoxypropyl, Methylthioäthyl, Äthylthioäthyl, Methylthiopropyl, Methylaminoäthyl, tert.-Butylaminoäthyl, Methoxyäthoxyäthyl, Äthoxyäthoxyäthyl.

Ein Carbamoylrest oder Thiocarbamoylrest trägt am Stickstoffatom entweder Wasserstoff oder ein oder zwei Reste aus der Gruppe Niederalkyl, Niederalkoxyalkyl, Niederalkenyl, Niederhaloalkenyl, Alkinyl oder ein Wasserstoffatom und wahlweise einen $C_3$-$C_6$-Cycloalkyl- oder aber einen Phenylring, der, wie für $R_1$, $R_2$ und $R_3$ angegeben, substituiert sein kann oder unsubstituiert ist. Beispiele für solche Reste sind Carbamoyl, Methylcarbamoyl, Äthylcarbamoyl, Cyclohexylcarbamoyl, n-Propylcarbamoyl, Anilido, 2,6-Dimethylanilido, 4-Chloranilido, 4-Methoxyanilido, 3-Trifluormethylanilido, Isopropylcarbamoyl, tert.-Butylcarbamoyl, Thioanilido, 4-Methylthioanilido.

Ein Sulfoxylrest besteht aus der Sulfoxygruppe und einem ggf. halogenierten $C_1$-$C_8$-Alkylrest oder einem Phenylrest, der gemäss $R_1$, $R_2$ und $R_3$ substituiert sein kann. Beispiele dafür sind Methylsulfonyl, Chlormethylsulfonyl, Trifluormethylsulfonyl, Phenylsulfonyl, 4-Chlorphenylsulfonyl, 4-Tolylsulfonyl, 4-Trifluormethylphenylsulfonyl, Trichlormethylsulfonyl.

Ein Sulfamoylrest trägt am Stickstoffatom entweder Wasserstoff oder bevorzugt zwei Reste aus der Gruppe Niederalkyl, Niederoxyalkyl, Niederalkenyl oder $C_4$-$C_5$-Alkylen, das durch Sauerstoff oder Schwefel unterbrochen sein kann. Beispiele dafür sind z. B. Dimethylsulfamoyl, Piperididosulfonyl, Morpholidosulfonyl.

Ein cyclischer Acylamidomethylrest besteht aus dem Dicarbonylimidomethylen Teil und einer Alkylen- halogenierten Alkylen-, Alkenylen- oder einer halogenierten Alkenylenkette. Beispiele dafür sind Malonylimidomethyl, Maloylimidomethyl, Succinylimidomethyl, Glutanylimidomethyl, Fomaroylimidomethyl, 2,3-Dimethylfumaroylimidomethyl, 2,3-Dichlorfumaroylimidomethyl.

Die Phthalimidomethylreste können sowohl im Phenylkern gemäss $R_1$, $R_2$ und $R_3$ substituiert wie auch teilweise oder ganz gesättigt, also 3,4-Dihydrophthalimidomethyl- und Tetrahydrophthalimidomethylreste sein.

Heterocyclische Reste sind mono- oder bicyclische Ringe mit 1 bis 3 gleichen oder verschiedenen Heteroatomen O, S und N. Zu nennen wären 3- bis 6-, vor allem 5- oder 6gliedrige Heterocyclen, die gesättigt, teilgesättigt oder ungesättigt und ggf. gemäss $R_1$, $R_2$ und $R_3$ substituiert sein können. Als Beispiele, die keine Limitierung darstellen sollen, seien genannt: Furan, Nitrofuran, Bromfuran, Methylfuran, Thiophen, Chlorthiophen, Pyridin, 2,6-Dichlorpyridin, Pyrimidin, Pyridazin, Pyrazin, Piperidin, Methylpiperidin, Morpholin, Thiomorpholin, Tetrahydrofuran, Oxazol, Pyrasol, Pyrrol, Pyrrolin, Pyrrolidin, Thiazol, 2,3-Dihydro-4H-pyran, Pyran, Dioxan, 1,4-Oxathia-(2)-in, Chinolin, Indol, Benzthiazol, Benzoxazol, Benzimidazol.

Denjenigen erfindungsgemässen Oximen kommt besondere Bedeutung zu, Verbindungen, welche der Formel Ia entsprechen

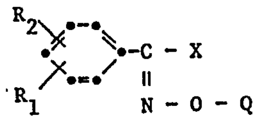
(Ia)

worin $R_1$, $R_2$, Q und X die oben gegebene Bedeutung haben. Dabei sind durch ihre Wirkung diejenigen Verbindungen besonders aufgefallen, in denen X die Trifluormethylgruppe bedeutet und welche der Formel Ib entsprechen

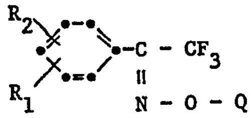
(Ib)

worin $R_1$, $R_2$ und Q die unter Formel I gegebene Bedeutung haben.

Bemerkenswert biologische Wirkung wurde mit den Oximäthern der Formel Ic erzielt

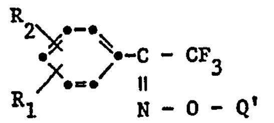
(Ic)

worin $R_1$ und $R_2$ die unter Formel I gegebene Bedeutung hat und Q' für $C_1$-$C_4$-Alkyl, insbesondere Methyl, Äthyl, Propyl und Butyl sowie Cyclopropyl, $C_3$-$C_4$-Alkenyl wie Allyl, $C_1$-$C_4$-Cyanoalkyl, wie Cyanomethyl und Cyanoäth-1-yl, Carbamoyl, $C_1$-$C_4$-Carbamoylalkyl wie Carbamoylmethyl oder Carbamoylät-1-yl.

Darüber fallen vor allem die Verbindungen:

– 1-Phenyl-1-methoximino-2,2,2-trifluoräthan,
– 1-(3-Trifluormethylphenyl)-1-cyclopropylmethoximino-2,2,2-trifluoäthan,
– 1-(4-Chlorphenyl)-1-n-propoximino-2,2,2-trifluoräthan,
– 1-(4-Chlorphenyl)-1-allyloximino-2,2,2-trifluoräthan,
– 1-Phenyl-1-carbamoylmethoximino-2,2,2-trifluoräthan,
– 1-(4-Fluorphenyl)-1-carbamoylmethoximino-2,2,2-trifluoräthan,
– 1-(4-Chlorphenyl)-1-carbamoylmethoximino-2,2,2-trifluoräthan,
– 1-(3-Trifluormethyl)-1-carbamoylmethoximino-2,2,2-trifluoräthan,
– 1-Phenyl-1-carbamoylmethoximino-2,2,3,3,3-pentafluorpropan,
– 1-Phenyl-1-carbamoyläth-1-oximino-2,2,2-trifluoräthan,
– 1-(4-Chlorphenyl)-1-carbamoyläth-1'-oximino-2,2,2-trifluoräthan,
– 1-(3-Trifluormethylphenyl)-1-carbamoyläth-1'-oximino-2,2,2-trifluoräthan,
– 1-(3-Chlorphenyl)-1-carbamoylmethoximino-2,2,2-trifluoräthan,
– 1-(3-Chlorphenyl)-1-isopropyloxycarbonylmethoximino-2,2,2-trifluoräthan.

Die Oximäther der Formel I werden erfindungsgemäss hergestellt, indem man ein Salz eines Oxims der Formel II

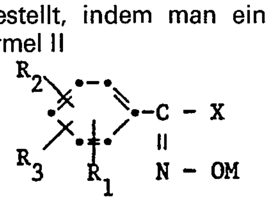
(II)

in welcher M ein Alkalimetall- oder Erdalkalimetallkation darstellt und $R_1$, $R_2$, $R_3$ und X die oben angegebene Bedeutung haben, mit einem reaktionsfähigen Ester der Formel III umsetzt

$$Y-Q \qquad \text{(III)}$$

worin Q die unter Formel I gegebene Bedeutung hat und Y einen organischen oder anorganischen Säurerest darstellt.

Als Salze eines Oxims der Formel II sind insbesondere die Natrium- und Kaliumsalze geeignet. Die Umsetzung des Oxims der Formel II mit dem reaktionsfähigen Ester der Formel III wird vorteilhaft in einem inerten organischen Lösungsmittel durchgeführt. Besonders geeignet sind polare Lösungsmittel, wie Acetonitril, Dimethylformamid, Dimethylacetamid, Methylpyrrolidon und Dimethylsulfoxid. Die Reaktanten werden in der Regel in äquimolarer Menge eingesetzt. Es kann jedoch auch zum vollständigen Ablauf der Reaktion, der eine oder der andere Reaktionspartner im Überschuss eingesetzt werden. Als reaktionsfähige Säurereste sind die Halogenide aber auch Sulfonsäurereste, z. B. von Methyl-, Äthyl-, Phenyl- oder Toluolsulfonsäure besonders geeignet. Die Umsetzung wird vorteilhafterweise bei einer Temperatur von 60-90° C durchgeführt. Wenn ein anderes Lösungsmittel, z. B. Toluol oder Chlorbenzol verwendet wird, so geschieht die Umsetzung bei höherer Temperatur und einer längeren Reaktionsdauer.

Gemäss der US-Patentschrift Nr. 4260555 wird der Rest Q mit einem Sulfonsäurerest verestert und der so erhaltenen Ester der Formel IV

$$Z-SO_3-Q \qquad \text{(IV)}$$

worin Z einen niederen Alkylrest oder einen durch Niederalkyl oder Halogen substituierten Phenylrest bedeutet mit dem Hydroxyoxim der Formel II oder einem Salz davon umsetzt.

Die Oxime der Formel II können in bekannter Weise durch Umsetzung der entsprechenden Ketone mit hydroxylamin hergestellt werden. Die hierfür benötigten Ketone können ihrerseits z. B. durch Umsetzung einer Grignard-Verbindung der Formel V

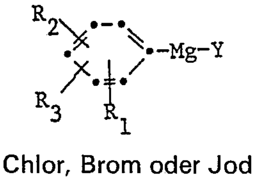
(V)

in welcher Y Chlor, Brom oder Jod bedeutet und $R_1$, $R_2$ und $R_3$ die oben angegebene Bedeutung haben, mit einer vom Rest X gemäss obiger Definition abgeleiteten Carbonsäure X–COOH, einem Salz davon, einem Säurechlorid X–COCl oder einem Nitril X–CN erhalten werden (vgl. US-Patent Nr. 3748361). Ferner ist es möglich, zur Herstellung der Oxime der Formel II geeignete Ketone durch Umsetzung eines Benzols der Formel VI

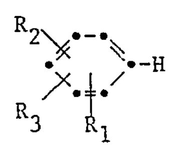
(VI)

in welcher $R_1$, $R_2$ und $R_3$ die oben angegebene Bedeutung haben, mit einem vom Rest X gemäss obiger Definition abgeleiteten Carbonsäurechlorid X–COCl in Gegenwart von Aluminiumchlorid zu gewinnen.

Oxime der Formel II, die zur Herstellung der neuen Oximäther der Formel I geeignet sind, sind beispielsweise:

– 1-Phenyl-1-hydroximino-2,2,2-trifluoräthan,
– 1-(4-Methylphenyl)-1-hydroximino-2,2,2-trifluoräthan,
– 1-(4-Chlorphenyl)-1-hydroximino-2,2,2-trifluoräthan,
– 1-(4-Fluorphenyl)-1-hydroximino-2,2,2-trifluoräthan,
– 1-(4-Trifluormethylphenyl)-1-hydroximino-2,2,2-trifluoräthan,
– 1-(3-Trifluormethylphenyl)-1-hydroximino-2,2,2-trifluoräthan,
– 1-(4-Methoxyphenyl)-1-hydroximino-2,2,2-trifluoräthan,
– 1-(4-Trifluormethoxyphenyl)-1-hydroximino-2,2,2-trifluoräthan,
– 1-(3-Nitrophenyl)-1-hydroximino-2,2,2-trifluoräthan,
– 1-(3,4-Dimethylphenyl)-1-hydroximino-2,2,2-trifluoräthan,
– 1-(3,4-Dichlorphenyl)-1-hydroximino-2,2,2-trifluoräthan,
– 1-(4-Chlorphenyl)-1-hydroximino-2,2-difluoräthan,
– 1-(4-Chlorphenyl)-1-hydroximino-2-chlor-2,2-difluoräthan,
– 1-(4-Methoxyphenyl)-1-hydroximino-2-chlor-2,2-difluoräthan,
– 1-(4-Trifluormethoxyphenyl)-1-hydroximino-2-chlor-2,2-difluoräthan,
– 1-(3-Nitrophenyl)-1-hydroximino-2-chlor-2,2-difluoräthan,
– 1-Phenyl-1-hydroximino-2,2,3,3,3-pentafluorpropan,
– 1-(4-Methylphenyl)-1-hydroximino-2,2,3,3,3-pentafluorpropan,
– 1-(4-Chlorphenyl)-1-hydroximino-2,2,3,3,3-pentafluorpropan,
– 1-(3-Nitrophenyl)-1-hydroximino-2,2,3,3,3-pentafluorpropan,
– 1-Phenyl-1-hydroximino-2,2,3,3,4,4,4-heptafluorbutan,
– 1-(4-Chlorphenyl)-1-hydroximino-2,2,3,3,4,4,4-heptafluorbutan,
– 1-(2-Chlorphenyl)-1-hydroximino-2,2,2-trifluoräthan,
– 1-(3-Chlorphenyl)-1-hydroximino-2,2,2-trifluoräthan.

Als reaktionsfähige Ester der Formel III kommen vor allem die Halogenide und Sulfonsäureester entsprechend der Definition von Q substituierter Niederalkylgruppen, niederen Alkenyl- oder Alkinyl- oder Cycloalkylgruppen in Frage. Ferner im Alkylteil halogenierte oder sulfonierte Alkancarbonsäurederivate. Ferner Arylhalogenide, Arylsulfonate, Aralkylhalogenide oder -sulfonate, ferner die Halide von Kohlensäureestern oder Thiokohlensäureestern, Alkyl- oder Arylsulfonylhalide, oder ein Halid einer Sulfamoylgruppe, wie auch die Halogenmethyl- oder Sulfonylmethylderivate von Acyliminomethyl oder Phthalimidomethylgruppen, ferner Halide oder Sulfonylester von heterocyclischen Resten, welche der Definition von Q entsprechen.

Solche Verbindungen sind bekannt oder können z. B. durch Umsetzen mit Halogenierungsmitteln wie Sulfurylchlorid oder -bromid, Thionylchlorid oder -bromid, Phosphoroxy- oder Phosphorylesterchlorid oder den entsprechenden Bromiden, einer konzentrierten Halogenwasserstoffsäure oder durch Umsetzen mit Halogeniden oder Anhydriden von Sulfonsäure hergestellt werden. Die Oximäther der Formel I besitzen in hervorragendem Masse die Eigenschaft, Kulturpflanzen vor Schädigung durch Agrarchemikalien zu schützen. Dieser Schutz erstreckt sich insbesondere auf Herbizide der verschiedenen Stoffklassen, darunter 1,3,5-Triazine, 1,2,4-Triazione, Phenylharnstoffderivate, Carbamate, Thiolcarbamate, Phenoxyessigsäureester, Phenoxypropionsäureester, Halogenacetanilide, Halogenphenoxyessigsäureester, substituierte Phenoxyphenoxyessigsäureester und -propionsäureester, substituierte Pyridyloxyphenoxyessigsäureester und -propionsäureester, Benzoesäurederivate usw., sofern diese nicht oder ungenügend kulturtolerant sind. Die Oximäther der Formel I sind vor allem zum Schützen von Kulturpflanzen gegen die schädigende Wirkung von Halogenacetaniliden und Thiolcarbamaten geeignet. Die Oximäther der Formel I können daher in bezug auf ihre Anwendung in Kombination mit den vorgenannten Herbiziden als Gegenmittel oder „Antidotes" oder auch als „Safener" bezeichnet werden.

Von den Verbindungen der Formel I existieren stereoisomere Formen, nämlich die syn- und anti-Formen der zugrundeliegenden Oxime und auch einzelne Enantiomere von solchen Verbindungen, die im Rest Q ein Asymmetriezentrum besitzen. Diese stereoisomeren Formen sind ebenfalls Gegenstand der Erfindung.

Ein solches Gegenmittel oder Antidote der Formel I kann je nach Anwendungszweck zur Vorbehandlung des Saatgutes der Kulturpflanze (Beizung des Samens oder der Stecklinge) eingesetzt oder vor oder nach der Saat in den Boden gegeben werden. Es kann aber auch für sich allein oder zusammen mit dem Herbizid vor oder nach dem Auflaufen der Pflanzen appliziert werden. Die Behandlung der Pflanze oder des Saatgutes mit dem Antidote kann daher grundsätzlich unabhängig vom Zeitpunkt der Applikation der phytotoxischen Chemikalie erfolgen. Die Behandlung der Pflanze kann jedoch auch durch gleichzeitige Applikation von phytotoxischer Chemikalie und Gegenmittel (Tankmischung) erfolgen. Die pre-emergente Behandlung schliesst sowohl die Behandlung der Anbaufläche vor der Aussaat (ppi = pre plant incorporation) als auch die Behandlung der angesäten, aber noch nicht bewachsenen Anbauflächen ein.

Die Aufwandmengen des Gegenmittels im Verhältnis zum Herbizid richten sich weitgehend nach

der Anwendungsart. Bei einer Feldbehandlung, bei der Herbizid und Gegenmittel entweder gleichzeitig (Tankmischung) oder separat appliziert werden, liegt das Verhältnis der Mengen von Gegenmittel zu Herbizid im Bereich von 1:100 bis 5:1. In der Regel wird bei einem Mengenverhältnis von Gegenmittel zu Herbizid von 1:5 bis 1:50 die volle Schutzwirkung erreicht. Bei der Samenbeizung und ähnlichen gezielten Schutzmassnahmen werden jedoch weit geringere Mengen Gegenmittel im Vergleich mit den später pro Hektar Anbaufläche verwendeten Mengen an Herbizid benötigt. Im allgemeinen werden bei der Samenbeizung pro kg Samen 0,1-10 g Gegenmittel benötigt. In der Regel wird mit 0,1-2 g Gegenmittel pro kg Samen bereits die volle Schutzwirkung erreicht. Falls das Gegenmittel kurz vor der Aussaat durch Samenquellung appliziert werden soll, so werden zweckmässig Lösungen des Gegenmittels, welche den Wirkstoff in einer Konzentration von 1-10000 ppm enthalten. In der Regel wird mit Konzentrationen des Gegenmittels von 100-1000 ppm die volle Schutzwirkung erreicht.

In der Regel liegt zwischen protektiven Massnahmen, wie Samenbeizung und Behandlung von Stecklingen mit einem Gegenmittel der Formel I und der möglichen späteren Feldbehandlung mit Agrarchemikalien ein längerer Zeitraum. Vorbehandeltes Saat- und Pflanzengut kann später in Landwirt, Gartenbau und Fortswirtschaft mit unterschiedlichen Chemikalien in Berührung kommen. Die Erfindung bezieht sich daher auch auf protektive Mittel für Kulturpflanzen, die als Wirkstoff ein Gegenmittel der Formel I zusammen mit üblichen Trägerstoffen enthalten. Solche Mittel können ggf. zusätzlich jene Agrarchemikalien enthalten, vor deren Einfluss die Kulturpflanze geschützt werden soll.

Als Kulturpflanzen gelten im Rahmen vorliegender Erfindung alle Pflanzen, die in irgendeiner Form Ertragsstoffe, wie Samen, Wurzeln, Stengel, Knollen, Blätter, Blüten, ferner Inhaltsstoffe, wie Öle, Zucker, Stärke, Eiweiss usw., produzieren und zu diesem Zweck angebaut werden. Zu diesen Pflanzen gehören beispielsweise sämtliche Getreidearten, wie Weizen, Roggen, Gerste und Hafer, daneben vor allem Reis, Kulturhirse, Mais, Baumwolle, Zuckerrüben, Zuckerrohr, Soja, Bohnen und Erbsen.

Das Gegenmittel kann überall dort eingesetzt werden, wo eine Kulturpflanze der vorgenannten Art vor der schädlichen Wirkung einer Agrarchemikalie geschützt werden soll. Dabei kommen als Agrarchemikalien, wie bereits ausgeführt, in erster Linie Herbizide der verschiedensten Stoffklassen, insbesondere jedoch Halogenacetanilide und Thiolcarbamate in Betracht.

Halogenacetanilide, deren schädigende Wirkung gegenüber Kulturpflanzen mit Hilfe der neuen Oximäther der Formel I aufgehoben werden kann, sind bereits in grosser Zahl bekannt geworden (vgl. z.B. DE-A Nrn. 2305495, 2328340, 2212268, 2726253 und 2805757 sowie US-Patentschriften Nrn. 3946044, 4022608 und 4039314). Solche Halogenacetanilide können durch die folgende allgemeine Formel VII beschrieben werden:

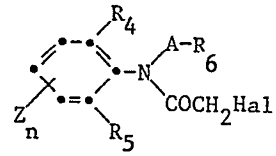

(VII)

In dieser Formel bedeuten Hal Halogen, insbesondere Chlor oder Brom, $R_4$ und $R_5$ unabhängig voneinander je Wasserstoff, Halogen, niederes Alkyl, Alkoxy, Alkylthio, Halogenalkyl, Alkoxyalkyl oder Alkylthioalkyl, Z Wasserstoff, Halogen, niederes Alkyl, Alkoxy, Alkylthio, Halogenalkyl, Alkoxyalkyl oder Alkylthioalkyl, wobei die vorgenannten Reste Z vorzugsweise in 3-Stellung in bezug auf das Stickstoffatom stehen, n 0 bis 3, A Alkylen, insbesondere Methylen, 1,1- und 1,2-Äthylen, wobei 1,2-Äthylen durch 1-2 niedere Alkylgruppen substituiert sein kann, und $R_6$ niederes Alkoxy, Hydroxycarbonyl, Alkoxycarbonyl, Carbamoyl, N-Alkylcarbamoyl, N,N-Dialkylcarbamoyl, Cyano, ein ggf. substituierter stickstoffhaltiger heterocyclischer Rest, Alkanoyl, ggf. substituiertes Benzoyl, ggf. substituiertes 1,3,4-Oxadiazol-2-yl, 1,3,4-Thiadiazol-2-yl, 1,3,4-Triazol-3-yl oder 1,3,4-Triazol-1-yl bedeutet.

Als einzelne Vertreter solcher Halogenacetanilide seien beispielsweise die folgenden genannt:

- N-Äthoxymethyl-N-chloracetyl-2-äthyl-6-methylanilin,
- N-Chloracetyl-N-methoxymethyl-2,6-diäthylanilin,
- N-Chloracetyl-N-(2-methoxyäthyl)-2,6-dimethylanilin,
- N-(2-Allyloxyäthyl)-N-chloracetyl-2,6-dimethylanilin,
- N-Chloracetyl-N-(2-n-propoxyäthyl)-2,6-dimethylanilin,
- N-Chloracetyl-N-(2-isopropoxyäthyl)-2,6-dimethylanilin,
- N-Chloracetyl-N-(2-methoxyäthyl)-2-äthyl-6-methylanilin,
- N-Chloracetyl-N-(methoxyäthyl)-2,6-diäthylanilin,
- N-(2-Äthoxyäthyl)-N-chloracetyl-2-äthyl-6-methylanilin,
- N-Chloracetyl-N-(2-methoxy-1-methyläthyl)-2-methylanilin,
- N-Chloracetyl-N-(2-methoxy-1-methyläthyl)-2,6-dimethylanilin,
- N-Chloracetyl-N-(2-methoxy-1-methyläthyl)-2,6-diäthylanilin,
- N-Chloracetyl-N-(2-methoxy-1-methyläthyl)-2-äthyl-6-methylanilin,
- N-(2-Äthoxyäthyl)-N-chloracetyl-2,6-diäthylanilin,
- N-Chloracetyl-N-(2-n-propoxyäthyl)-2-äthyl-6-methylanilin,
- N-Chloracetyl-N-(2-n-propoxyäthyl)-2,6-diäthylanilin,
- N-Chloracetyl-N-(2-isopropoxyäthyl)-2-äthyl-6-methylanilin,

– N-Äthoxycarbonylmethyl-N-chloracetyl-2,6-dimethylanilin,
– N-Äthoxycarbonylmethyl-N-chloracetyl-2,6-diäthylanilin,
– N-Chloracetyl-N-methoxycarbonylmethyl-2,6-dimethylanilin,
– N-Chloracetyl-N-(2,2-diäthoxyäthyl)-2,6-dimethylanilin,
– N-Chloracetyl-N-(2-methoxy-1-methyläthyl)-2,3-dimethylanilin,
– N-(2-Äthoxyäthyl)-N-chloracetyl-2-methylanilin,
– N-Chloracetyl-N-(2-methoxyäthyl)-2-methylanilin,
– N-Chloracetyl-N-(2-methoxy-2-methyläthyl)-2,6-dimethylanilin,
– N-(2-Äthoxy-2-methyläthyl)-N-chloracetyl-2-äthyl-6-methylanilin,
– N-Chloracetyl-N-(1-äthyl-2-methoxyäthyl)-2,6-dimethylanilin,
– N-Chloracetyl-N-(2-methoxyäthyl)-2-methoxy-6-methylanilin,
– N-n-Butoxymethyl-N-chloracetyl-2-tert.-butylanilin,
– N-(2-Äthoxyäthyl-1-methyläthyl)-2,6-dimethylanilin,
– N-Chloracetyl-N-(2-methoxyäthyl)-2-chlor-6-methylanilin,
– N-(2-Äthoxyäthyl)-N-chloracetyl-2-chlor-6-methylanilin,
– N-(2-Äthoxyäthyl)-N-chloracetyl-2,3,6-trimethylanilin,
– N-Chloracetyl-1-(2-methoxyäthyl)-2,3-6-trimethylanilin,
– N-Chloracetyl-N-cyanomethyl-2,6-dimethylanilin,
– N-But-3-in-1-yl-N-chloracetylanilin,
– N-Chloracetyl-N-propargyl-2-äthyl-6-methylanilin,
– N-Chloracetyl-N-(1,3-dioxolan-2-ylmethyl)-2,6-dimethylanilin,
– N-Chloracetyl-N-(1,3-dioxolan-2-ylmethyl)-2-äthyl-6-methylanilin,
– N-Chloracetyl-N-(1,3-dioxan-2-ylmethyl)-2-äthyl-6-methylanilin,
– N-Chloracetyl-N-(2-furanylmethyl)-2,6-dimethylanilin,
– N-Chloracetyl-N-(2-furanylmethyl)-2-äthyl-6-methylanilin,
– N-Chloracetyl-N-(2-tetrahydrofuranylmethyl)-2,6-dimethylanilin,
– N-Chloracetyl-N-(N-propargylcarbamoylmethyl)-2,6-dimethylanilin,
– N-Chloracetyl-N-(N,N-dimethylcarbamoylmethyl)-2,6-dimethylanilin,
– N-(n-Butoxymethyl)-N-chloracetyl-2,6-diäthylanilin,
– N-(2-n-Butoxyäthyl)-N-chloracetyl-2,6-diäthylanilin,
– N-Chloracetyl-N-(2-methoxy-1,2-dimethyläthyl)-2,6-dimethylanilin,
– N-Chloracetyl-N-isopropyl-2,3-dimethylanilin,
– N-Chloracetyl-N-isopropyl-2-chloranilin,
– N-Chloracetyl-N-(1H-pyrazol-1-ylmethyl)-2,6-dimethylanilin,
– N-Chloracetyl-N-(1H-pyrazol-1-ylmethyl)-2-äthyl-6-methylanilin,
– N-Chloracetyl-N-(1H-1,2,4-triazol-1-ylmethyl)-2,6-dimethylanilin,
– N-Chloracetyl-N-(1H-1,2,4-triazol-1-ylmethyl)-2,6-diäthylanilin,
– N-Benzoylmethyl-N-chloracetyl-2,6-dimethylanilin,
– N-Benzoylmethyl-N-chloracetyl-2-äthyl-6-methylanilin,
– N-Chloracetyl-N-(5-methyl-1,3,4-oxadiazol-2-yl)-2,6-diäthylanilin,
– N-Chloracetyl-N-(5-methyl-1,3,4-oxadiazol-2-yl)-2-äthyl-6-methylanilin,
– N-Chloracetyl-N-(5-methyl-1,3,4-oxadiazol-2-yl)-2-tert.-butylanilin,
– N-Chloracetyl-N-(4-chlorbenzoylmethyl)-2,6-dimethylanilin,
– N-Chloracetyl-N-(1-methyl-5-methylthio-1,3,4-triazol-2-ylmethyl)-2,6-diäthylanilin.

Weitere Halogenacetanilide, deren schädigende Wirkung auf Kulturpflanzen durch die Oximäther der Formel I aufgehoben werden kann, sind in R. Wegler, „Chemie der Pflanzenschutz- und Schädlingsbekämpfungsmittel", Bd. 8, S. 90-93 und S. 322-327 aufgeführt.

Herbizide wirksame Thiolcarbamate, vor deren phytotoxischer Wirkung Kulturpflanzen durch die neuen Oximäther der Formel I geschützt werden können, sind ebenfalls bereits in grosser Zahl bekannt geworden (vgl. z.B. US-Patentschriften Nrn. 2913327, 3037853, 3175897, 3185720, 3198786, 3582314 und 3846115). Die Schutzwirkung der neuen Oximäther der Formel I lässt sich insbesondere beim Einsatz von Thiolcarbamaten in Getreide, Reis oder veredelter Sorghum-Hirse vorteilhaft ausnützen.

Die Thiolcarbamate, vor deren phytotoxischer Wirkung Kulturpflanzen, wie Getreide, Reis und veredelte Sorghum-Hirse bevorzugt geschützt werden können, entsprechen den allgemeinen Formeln VIII und IX:

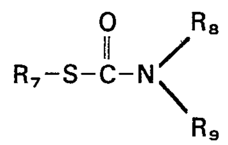
(VIII)

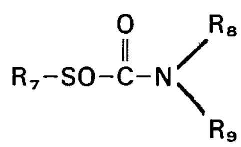
(IX)

In diesen Formeln bedeutet $R_7$ niederes Alkyl, Alkenyl, Chlorallyl, Dichlorallyl, Trichlorallyl, Benzyl oder 4-Chlorbenzyl, $R_8$ $C_2$-$C_4$-Alkyl und $R_9$ $C_2$-$C_4$-Alkyl oder Cyclohexyl, wobei die Reste $R_8$ und $R_9$ zusammen mit dem Stickstoffatom, an das sie gebunden sind, einen Hexahydro-1H-azepin-, Dekahydrochinolin- oder 2-Methyldekahydrochinolinring bilden können.

Als einzelne Vertreter solcher Thiolcarbamate seien beispielsweise die folgenden genannt:
– S-Äthyl-N,N-dipropylthiocarbamat,

- S-Äthyl-N,N-diisobutylthiocarbamat,
- S-2,3-Dichlorallyl-N,N-diisopropylthiolcarbamat,
- S-Propyl-N-butyl-N-äthylthiolcarbamat,
- S-2,3,3-Trichlorallyl-N,N-diisopropylthiocarbamat,
- S-Propyl-N,N-dipropylthiolcarbamat,
- S-Äthyl-N-äthyl-N-cyclohexylthiolcarbamat,
- S-Äthyl-N-hexahydro-1H-azepin-1-carbothioat,
- S-Isopropyl-N,N-hexamethylenthiolcarbamat,
- S-(p-Chlorbenzyl)-N,N-diäthylthiolcarbamat,
- N-Äthylthiocarbonyl-cis-decahydrochinolin,
- N-Propylthiocarbonyldecahydrochinaldin,
- S-Äthyl-N,N-bis(n-butyl)-thiolcarbamat,
- S-tert.-Butyl-N,N-bis(n-propyl)-thiolcarbamat.

Die angewendete Menge der Gegenmittel schwankt zwischen etwa 0,01 und etwa 15 Gew.-Teilen pro Gewichtsteil Herbizide. Man ermittelt von Fall zu Fall, d. h, je nach verwendetem Herbizid-Typ, welches Verhältnis in bezug auf optimale Wirkung bei der speziellen Kulturpflanze das geeignetste ist.

Die Verbindungen der Formel I werden in unveränderter Form oder vorzugsweise zusammen mit den in der Formulierungstechnik üblichen Hilfsmitteln eingesetzt und werden daher z. B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z. B. polymeren Stoffen in bekannter Weise verarbeitet. Die Anwendungsverfahren wie Versprühen, Vernebeln, Verstäuben, Verstreuen oder Giessen werden gleich wie die Art der Mittel den angestrebten Zielen und den gegebenen Verhältnissen entsprechend gewählt.

Die Formulierungen d. h. die den Wirkstoff der Formel I und ggf. einen festen oder flüssigen Zusatzstoff enthaltenden Mittel, Zubereitungen oder Zusammensetzungen werden in bekannter Weise hergestellt, z.B. durch inniges Vermischen und/oder Vermahlen der Wirkstoffe mit Streckmitteln, wie z.B. mit Lösungsmitteln, festen Trägerstoffen, und ggf. oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z. B. Xylolgemische oder substituierte Naphthaline, Phthalsäureester wie Dibutyl- oder Di-octylphthalat, aliphatische Kohlenwasserstoffe wie Cyclohexan oder Paraffine, Alkohole und Glykole sowie deren Äther und Ester, wie Äthanol, Äthylenglykol, Äthylenglykolmonomethyl- oder -äthyläther, Ketone wie Cyclohexanon, stark polare Lösungsmittel wie N-Methyl-2-pyrrolidon, Dimethylsulfoxid oder Dimethylformamid, sowie ggf. epoxydierte Pflanzenöle wie epoxydiertes Kokosnussöl oder Sojaöl; oder Wasser.

Als feste Trägerstoffe, z. B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z. B. Bimsstein, Ziegelbruch, Sepiolit oder Bentonit, als nicht sorptive Trägermaterialien z. B. Calcit oder Sand in Frage. Darüber hinaus kann eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen je nach der Art des zu formulierenden Wirkstoffes der Formel I nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen wie wasserlösliche synthetische oberflächenaktive Verbindungen sein.

Als Seifen sind z. B. die Alkali-, Erdalkali- oder ggf. substituierten Ammoniumsalze von höheren Fettsäuren ($C_{10}$-$C_{22}$), wie z. B. die Na- oder K-Salze der Öl- oder Stearinsäure, oder von natürlichen Fettsäuregemischen, die z. B. aus Kokosnuss- oder Talgöl gewonnen werden können. Ferner sind auch die Fettsäuremethyltaurinsalze zu erwähnen.

Häufiger werden jedoch sog. synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali oder ggf. substituierte Ammoniumsalze vor und weisen einen Alkylrest mit 8 bis 22 C-Atomen auf, wobei Alkyl auch den Alkylteil von Acylresten einschliesst, z. B. das Na- der Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Äthylenoxyd-Addukten. Die sulfonierten Benzimidazolderivate enthalten vorzugsweise 2 Sulfonsäuregruppen und einen Fettsäurerest mit 8 bis 22 C-Atomen. Alkylarylsulfonate sind z.B. die Na-, Ca- oder Triäthanolaminsalze der Dodecylbenzolsulfonsäure, der Dibutylnaphthalinsulfonsäure, oder eines Naphthalinsulfonsäure-Formaldehydkondensationsproduktes.

Ferner kommen auch entsprechende Phosphate wie z. B. Salze des Phosphorsäureesters eines p-Nonylphenol-(4-14)-Äthylenoxyd-Adduktes in Frage.

Als nichtionisches Tensid kommen in erster Linie Polyglykolätherderivate von aliphatischen oder cycloaliphatischen Alkoholen, gesättigten oder ungesättigten Fettsäuren und Alkylphenolen in Frage, die 3 bis 30 Glykoläthergruppen und 8 bis 20 Kohlenstoffatome im (aliphatischen) Kohlenwasserstoffrest und 6 bis 18 Kohlenstoffatome im Alkylrest der Alkylphenol enthalten können.

Weitere geeignete nichtionische Tenside sind die wasserlöslichen, 20 bis 250 Äthylenglykoläthergruppen und 10 bis 1000 Propylenglykoläthergruppen enthaltenden Polyäthylenoxidaddukte an Polypropylenglykol, Äthylendiaminopolypropylenglykol und Alkylpolypropylenglykol mit 1 bis 10 Kohlenstoffatomen in der Alkylkette. Die genannten Verbindungen enthalten üblicherweise pro Propylenglykoleinheit 1 bis 5 Äthylenglykoleinheiten.

Als Beispiele nichtionischer Tenside seien Nonylphenolpolyäthoxyäthanole, Ricinusölpolyglykoläther, Polypropylen-Polyäthylenoxyaddukte, Tributylphenoxypolyäthoxyäthanol, Polyäthylenglykol und Octylphenoxypolyäthoxyäthanol erwähnt.

Ferner kommen auch Fettsäureester von Polyoxyäthylensorbitan wie das Polyoxyäthylensorbitantrioleat in Betracht.

Bei den kationischen Tensiden handelt es sich vor allem um quartäre Ammoniumsalze, welche als N-Substituenten mindestens einen Alkylrest mit 8 bis 22 C-Atomen enthalten und als weitere Substituenten niedrige, ggf. halogenierte Alkyl-, Benzyl- oder niedrige Hydroxyalkylreste aufweisen. Die Salze liegen vorzugsweise als Halogenide, Methylsulfate oder Äthylsulfate vor, z.B. das Stearyltrimethylammoniumchlorid oder das Benzyldi-(2-chloräthyl)äthylammoniumbromid.

Die in der Formulierungstechnik gebräuchlichen Tenside sind u. a. in folgenden Publikationen beschrieben:

„Mc Cutcheon's Detergents and Emulsifiers Annual", Mc Publishing Corp., Ringwood, New Jersey, 1979.

Sisely and Wood, „Encyclopedia of Surface Active Agents", Chemical Publishing Co., Inc. New York, 1964.

Die pestiziden Zubereitungen enthalten in der Regel 0,1 bis 95%, insbesondere 0,1 bis 80%, Wirkstoff der Formel I, 1 bis 99,9% eines festen oder flüssigen Zusatzstoffes und 0 bis 25%, insbesondere 0,1 bis 25% eines Tensides.

Insbesondere setzen sich bevorzugte Formulierungen folgendermassen zusammen: (% = Gewichtsprozent)

*Emulgierbare Konzentrate*

Aktiver Wirkstoff: 1 bis 20%, bevorzugt 5 bis 10%
oberflächenaktives Mittel: 5 bis 30%, vorzugsweise 10 bis 20%
flüssiges Trägermittel: 50 bis 94%, vorzugsweise 70 bis 85%

*Stäube*

Aktiver Wirkstoff: 0,1 bis 10%, vorzugsweise 0,1 bis 1%
festes Trägermittel: 99,9 bis 90%, vorzugsweise 99,9 bis 99%

*Suspension-Konzentrate*

Aktiver Wirkstoff: 5 bis 75%, vorzugsweise 10 bis 50%
Wasser: 94 bis 25%, vorzugsweise 90 bis 30%
oberflächenaktives Mittel: 1 bis 40%, vorzugsweise 2 bis 30%

*Benetzbare Pulver*

Aktiver Wirkstoff: 0,5 bis 90%, vorzugsweise 1 bis 80%
oberflächenaktives Mittel: 0,5 bis 20%, vorzugsweise 1 bis 15%
festes Trägermittel: 5 bis 95%, vorzugsweise 15 bis 90%

*Granulate*

Aktiver Wirkstoff: 0,5 bis 30%, vorzugsweise 3 bis 15%
festes Trägermittel: 99,5 bis 70%, vorzugsweise 97 bis 85%

Während als Handelsware eher konzentrierte Mittel bevorzugt werden, verwendet der Endverbraucher in der Regel verdünnte Mittel. Die Anwendungsformen können bis hinab zu 0,001% an Wirkstoff verdünnt werden. Die Aufwandmengen betragen in der Regel 0,01 bis 10 kg AS/ha, vorzugsweise 0,025 bis 5 kg AS/ha.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

In den folgenden Beispielen sind die Temperaturen in Celsiusgraden °C, die Drücke in Millibar mb angegeben.

*Beispiel 1:*

Herstellung von 1-Phenyl-1-cyanomethoximino-2,2,2-trifluoräthan (Verbindung Nr. 4)

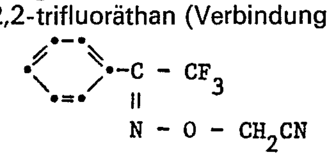

In eine Lösung von 2,3 g (0,1 mol) metallisches Natrium in 50 ml absolutem Äthanol werden 19 g (0,1 mol) 1-(Hydroximino)-1-phenyl-2,2,2-trifluoräthan eingerührt und das Lösungsmittel abgedampft. Der Rückstand wird in 50 ml Dimethylsulfoxid gelöst und dazu 15,1 g (0,2 mol) Chloracetonitril getropft. Nach beendigter Zugabe des Chloracetonitrils wird zunächst 4 h bei 60 bis 70° Innentemperatur nachgerührt und darauf das Reaktionsgemisch auf Eis/Wasser gegossen. Die organische Phase wird mit Methylenchlorid extrahiert. Nach Trocknen des Extraktes und Eindampfen des Lösungsmittels erhält man 12,1 g kristallines Titelprodukt (53,1% der Theorie), welches nach Umkristallisieren aus Isopropanol einen Schmelzpunkt von 54 bis 56° aufweist.

*Analyse:*

| | | | | |
|---|---|---|---|---|
| Ber.: | C 52,64 | H 3,09 | N 12,38 | F 24,98% |
| Gef.: | C 52,3 | H 3,2 | N 12,4 | F 24,4 % |

In analoger Weise zu diesem Beispiel werden folgende Verbindungen hergestellt:

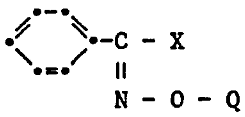

| Nr. | Phenylsubstitution | X | Q | phys. Daten |
|---|---|---|---|---|
| 1 | — | $CF_3$ | $CH_3$ | Sdp. 30-31°/0,08 mbar |
| 2 | — | $CF_3$ | $C_3H_7n$ | Sdp. 40-41°/0,08 mbar |
| 3 | — | $CF_3$ | $CH_2C{\equiv}CH$ | Sdp. 50-54°/0,03 mbar |
| 4 | — | $CF_3$ | $CH_2CN$ | Smp. 54-56° |
| 5 | — | $CF_3$ | $CH_2CONH_2$ | Smp. 84-86° |
| 6 | — | $CF_3$ | $CON(CH_3)CH_2CN$ | $n_D^{20}$ = 1,4960 |
| 7 | 4-Cl | $CHF_2$ | $CH(CH_3)COOC_2H_5$ | |
| 8 | — | $CF_3$ | $COC(Cl)=C(Cl)_2$ | $n_D^{20}$ = 1,5210 |
| 9 | 4-Cl | $CF_3$ | $SO_2CF_3$ | |
| 10 | — | $CF_3$ | $CH_2OCOC(CH_3)_3$ | Sdp. 79-80°/0,07 mbar |
| 11 | 4-F | $CF_3$ | $CH_3$ | Sdp. 63-64°/14 mbar |
| 12 | 4-F | $CF_3$ | $C_3H_7n$ | Sdp. 82-84°/12 mbar |
| 13 | 4-F | $CF_3$ | $CH_2CN$ | Sdp. 119-122°/12 mbar |
| 14 | 4-F | $CF_3$ | $CH_2CONH_2$ | Smp. 52-54° |
| 15 | 4-Cl | $CF_3$ | $CON(CH_3)CH_2CN$ | wachsartig |
| 16 | 4-Cl | $CF_3$ | $CON(CH_3)C_2H_4CN$ | wachsartig |
| 17 | 4-Cl | $CF_3$ | $COC_6H_4CH_3(2)$ | $n_D^{20}$ = 1,5155 |
| 18 | 4-Cl | $CF_3$ | $COC_6H_4CF_3(3)$ | $n_D^{20}$ = 1,4900 |
| 19 | 4-Cl | $CF_3$ | $SO_2CHCl$ | |
| 20 | 4-Cl | $CF_3$ | $CH_2COOC_3H_7$ iso | Sdp. 90-91°/0,09 mbar |
| 21 | 4-Cl | $CF_3$ | $CH_2CN$ | Öl $n_D^{24}$ = 1,4930 |
| 22 | 4-Cl | $CF_3$ | $COC_6H_5$ | |
| 23 | 3-Cl | $CClF_2$ | $CH_3$ | |
| 24 | — | $CClF_2$ | $C_3H_{7n}$ | |
| 25 | 4-Br | $CClF_2$ | $CH_2CH=CH_2$ | |
| 26 | $4\text{-}NO_2$ | $CClF_2$ | $CH_3$ | |
| 27 | $4\text{-}CF_3$ | $CF_3$ | $CH_2CN$ | |
| 28 | $4\text{-}OCF_3$ | $CF_3$ | $CH_3$ | |
| 29 | $4\text{-}C_4H_9$ | $CF_3$ | $CH_2CN$ | |
| 30 | — | $C_2F_5$ | $COC_6H_4Cl(4)$ | |
| 31 | — | $C_2F_5$ | $CH_3$ | |
| 32 | — | $C_2F_5$ | $CONH_2$ | |
| 33 | — | $C_2F_5$ | $CH_2CN$ | |
| 34 | 4-Cl | $C_2F_5$ | $CH_3$ | |
| 35 | 4-Cl | $C_2F_5$ | $CH_3$ | |
| 36 | 4-Cl | $C_2F_5$ | $COCH_3$ | |
| 37 | $4\text{-}OCH_3$ | $CF_3$ | $CH_3$ | |
| 38 | $4\text{-}OCH_3$ | $CF_3$ | $C_3H_{7n}$ | |
| 39 | $4\text{-}OCH_3$ | $CF_3$ | $CH_2-CH=CH_2$ | |
| 40 | $4\text{-}OCH_3$ | $CF_3$ | $CH_2CN$ | |
| 41 | $4\text{-}OCF_3$ | $CF_3$ | $CH_3$ | |
| 42 | $4\text{-}OCF_3$ | $CF_3$ | $CH_2CN$ | |
| 43 | $4\text{-}OCF_3$ | $CF_2Cl$ | $C_3H_{7n}$ | |
| 44 | 3-Cl | $CF_3$ | $CH_2$ | |
| 45 | 2-Cl | $CF_3$ | $CH_2CN$ | 73-74°/0,1 |
| 46 | 3-Cl | $CF_3$ | $CH_2CONH_2$ | |
| 47 | 3-Cl | $CF_3$ | $CH_2-CH=CH_2$ | |
| 48 | $3\text{-}NO_2$ | $CF_3$ | $CH_2CN$ | |
| 49 | $3\text{-}NO_2$ | $CF_3$ | $CH_2CONH_2$ | |
| 50 | $3\text{-}NO_2$ | $CF_3$ | $CH_2C(CH_3)_3$ | |
| 51 | $3\text{-}NO_2$ | $CF_3$ | $CH_2-CH=CH_2$ | |
| 52 | 2-Cl | $CF_3$ | $C_3H_7n$ | |
| 53 | 2-Cl | $CF_3$ | $C_2H_4CN$ | |
| 54 | 2-Cl | $CF_3$ | $CH_2-CCl=C(Cl)_2$ | |
| 55 | 4-Cl | $CF_3$ | $CH_2CONHNH_2$ | |
| 56 | 4-Br | $CF_3$ | $C_3H_7i$ | |
| 57 | 4-Br | $CF_3$ | $CH_2C(CH_3)_3$ | |

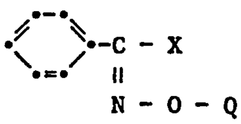

| Nr. | Phenylsubstitution | X | Q | phys. Daten |
|---|---|---|---|---|
| 58 | 4-Br | $CF_3$ | $CH_2CN$ | |
| 59 | 4-Br | $CF_3$ | $CH_2CONH_2$ | |
| 60 | 4-$CF_3$ | $CF_3$ | $CH_2CONHCH_3$ | |
| 61 | 4-$CF_3$ | $CF_3$ | $CH(CH_3)CN$ | |
| 62 | 4-$CF_3$ | $CF_3$ | $CH_2CONH_2$ | |
| 63 | 4-$CF_3$ | $CF_3$ | $CH_2C(CH_3)=CH_2$ | |
| 64 | 4-Cl | $CF_3$ | $C_2H_4-COOC_2H_5$ | |
| 65 | 4-$OCHF_2$ | $CF_3$ | $CH_2CN$ | |
| 66 | 4-$OCHF_2$ | $CF_3$ | $CH_2COOCH_3$ | |
| 67 | 4-$OCHF_2$ | $CF_3$ | $CH_2CONH_2$ | |
| 68 | — | $CF_3$ | $CH_2-CH_2-N(CH_3)_2$ | 57-57°/0,08 |
| 69 | — | $CF_3$ | 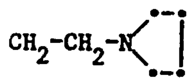 | 87-90°/0,09 |
| 70 | — | $CF_3$ | $CH_2-CH=CH_2$ | |
| 71 | — | $CF_3$ | $CH_2-CH=CH-CH_3$ | 55-57°/0,1 |
| 72 | — | $CF_3$ | $CH(CH_3)CO-NH_2$ | $n_D^{24}$ = 1,4950 |
| 73 | 4-F | $CF_3$ | $CH_2-CH=CH_2$ | 38-40°/0,15 |
| 74 | 4-F | $CF_3$ | $CH_2-C\equiv CH$ | 55-56°/0,9 |
| 75 | 4-Cl | $CF_3$ | $CH(CH_3)CONHNH_2$ | |
| 76 | 4-Cl | $CHF_2$ | Cyclopropylmethyl | |
| 77 | 4-Cl | $CF_3$ | $CH_2CH_2COOC_2H_5$ | |
| 78 | 4-F | $CF_3$ | $CO-N(CH_3)CH_2-CN$ | |
| 79 | 4-Cl | $CF_3$ | $CH(C_2H_5)CONH_2$ | |
| 80 | 4-Cl | $CF_3$ | $CH_3$ | 80°/19 |
| 81 | 4-Cl | $CF_3$ | $C_3H_7(n)$ | Öl |
| 82 | 4-Cl | $CF_3$ | $CH_2-C\equiv CH$ | 55-59°/0,2 |
| 83 | 4-Cl | $CF_3$ | $CH_2-CH=CH_2$ | 53-56°/0,03 |
| 84 | 4-Cl | $CF_3$ | $CH_2-CO-NH_2$ | Smp. 63-65° |
| 85 | 4-Cl | $CF_3$ | $CH(CH_3)CO-NH_2$ | Smp. 79-81° |
| 86 | 4-Cl | $CF_3$ | 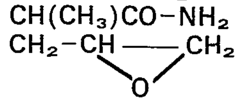 | |
| 87 | 4-Cl | $CF_3$ | $CH_2-CH_2-N(CH_3)_2$ | |
| 88 | 4-Cl | $CF_3$ | 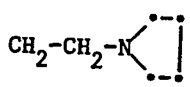 | |
| 89 | 3-Cl | $CF_3$ | $CH_2COOC_6H_5$ | |
| 90 | 3-Cl | $CF_3$ | $CH_2COOC(CH_3)_2$ | |
| 91 | 3-Cl | $CF_3$ | $CH_2COOCH_2CH=CH_2$ | |
| 92 | 3,4-$Cl_2$ | $CF_3$ | $CH(CH_3)COONa$ | |
| 93 | 3,4-$Cl_2$ | $CF_2Cl$ | $CH(CH_3)COOK$ | |
| 94 | 3,4-$Cl_2$ | $CF_3$ | $CH(CH_3)CH=CHCOOCH_3$ | |
| 95 | 3-$NO_2$ | $CF_3$ | $CH_2CH=CHCOOCH_3$ | |
| 96 | 3-$NO_2$ | $CF_3$ | $CH(CH_3)CH=CHCOSCH_3$ | |
| 97 | 3-$NO_2$ | $CF_3$ | $CH_2COOCH_2CN$ | |
| 98 | 3-$NO_2$ | $CF_3$ | $CH_2COSCH_2CH=CH_2$ | |
| 99 | 3-$NO_2$ | $CF_3$ | $CH_2COOC_6H_5$ | |
| 100 | 3-$NO_2$ | $CF_3$ | $CH_2COSCH_2C\equiv CH$ | |
| 101 | 4-Cl | $CF_3$ | $CH_2-CH=CHCOSCH_3$ | |
| 102 | 4-Cl | $CF_3$ | $CH_2-CH=CHCOOCH_3$ | |
| 103 | 3,4-$Cl_2$ | $CF_3$ | $CH_2COOCH_2CH\equiv CH$ | |
| 104 | 3,4-$Cl_2$ | $CF_3$ | $CH_2CONHCHO$ | |
| 105 | 3,4-$Cl_2$ | $CF_3$ | $CH_2CONH-NH_2$ | |
| 106 | 3,4-$Cl_2$ | $CF_3$ | $CH(CH_3)NH-NH_2$ | |
| 107 | 3,4-$Cl_2$ | $CF_3$ | $CH_2-CSNHCH_2-CH=CH_2$ | |
| 108 | 4-$CH_3$ | $CF_3$ | $CH_2-CONHCONHC_6H_5$ | |

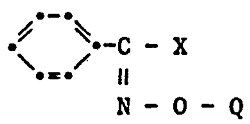

| Nr. | Phenylsubstitution | X | Q | phys. Daten |
|---|---|---|---|---|
| 109 | 4-$CH_3$ | $CF_3$ | $CH_2$–$CONHCONHC_6H_4Cl$(4) | |
| 110 | 4-$CH_3$ | $CF_3$ | $CH(CH_3)CONHCON(CH_3)_2$ | |
| 111 | 4-$CH_3$ | $CF_3$ | $CH_2CONHNH_2$ | |
| 112 | 4-$OCH_3$ | $CF_2Cl$ | $COCH_3$ | |
| 113 | 4-$OCH_3$ | $CF_2Cl$ | $COC_2H_5$ | |
| 114 | 4-$OCH_3$ | $CF_2Cl$ | $COC_3H_7$n | |
| 115 | 4-$OCH_3$ | $CF_2Cl$ | $COC_4H_9$t | |
| 116 | 4-$OCH_3$ | $CF_2Cl$ | $COC_3H_7$i | |
| 117 | 4-$OCH_3$ | $CF_2Cl$ | $COCH=C(CH_3)_2$ | |
| 118 | 2-Cl, 4-$CH_3$ | $CF_3$ | $COCH_2Cl$ | |
| 119 | 2-Cl, 4-$CH_3$ | $CF_3$ | $COC(CH_3)_2CH_2Cl$ | |
| 120 | 2-Cl, 4-$CH_3$ | $CF_3$ | $COCF_3$ | |
| 121 | 2-Cl, 4-$CH_3$ | $CF_3$ | $COC(Cl)=CCl_2$ | |
| 122 | 2-Cl, 4-$CH_3$ | $CF_3$ | $COCOOC_2H_5$ | |
| 123 | 2-Cl, 4-$CH_3$ | $CF_3$ | $COCOOC_2H_5$ | |
| 124 | 2-Cl, 4-$CH_3$ | $CF_3$ | $COCONH_2$ | |
| 125 | 2-Cl, 4-$CH_3$ | $CF_3$ | $COCH_2OCH_3$ | |
| 126 | 2-Cl, 4-$CH_3$ | $CF_3$ | $COCH_2OC_6H_5$ | |
| 127 | 2-Cl, 4-$CH_3$ | $CF_3$ | $COCH(CH_3)OC_6H_5$ | |
| 128 | 2-Cl, 4-$CH_3$ | $CF_3$ | $COCH(CH_3)OC_6H_4Cl$(4) | |
| 129 | 2-Cl, 4-$CH_3$ | $CF_3$ | $COCH(CH_3)OC_6H_4Cl$(2) | |
| 130 | 2-Cl, 4-$CH_3$ | $CF_3$ | $COCH_2OC_6H_3Cl_2$(2,4) | |
| 131 | 2-Cl, 4-$CH_3$ | $CF_3$ | $COCH(CH_3)C_6H_3Cl(2)CH_3(4)$ | |
| 132 | 2-Cl, 4-$CH_3$ | $CF_3$ | $COCH_2SC_6H_5$ | |
| 133 | 2-Cl, 4-$CH_3$ | $CF_3$ | $COCH(CH_3)SC_6H_5Cl$(4) | |
| 134 | 2-Cl, 4-$CH_3$ | $CF_3$ | $COCH_3C_6H_5$ | |
| 135 | 2-Cl, 4-$CH_3$ | $CF_3$ | $COCH_2C_6H_4Cl$ | |
| 136 | 2-Cl, 4-$CH_3$ | $CF_3$ | $COCH(C_3H_7i)C_6H_5$ | |
| 137 | 2-Cl, 4-$CH_3$ | $CF_3$ | $COCH(C_3H_7i)C_3H_4Cl$(4) | |
| 138 | 2-Cl, 4-$CH_3$ | $CF_3$ | CO-Cyclopropyl | |
| 139 | 2-Cl, 4-$CH_3$ | $CF_3$ | CO-1-Methylcyclopropyl | |
| 140 | 2-Cl, 4-$CH_3$ | $CF_3$ | CO-2,2-Dimethylcyclopropyl | |
| 141 | 2-Cl, 4-$CH_3$ | $CF_3$ | CO-2,2-Dichlorcyclopropyl | |
| 142 | 2-Cl, 4-$CH_3$ | $CF_3$ | CO-Cyclopentyl | |
| 143 | 2-Cl, 4-$CH_3$ | $CF_3$ | CO-Cyclohexyl | |
| 144 | 2-Br | $CF_2Cl$ | $COC_6H_5$ | |
| 145 | 2-Br | $CF_2Cl$ | $COC_6H_4(CH_3)$(2) | |
| 146 | 2-Br | $CF_2Cl$ | $COC_6H_4(CH_3)$(3) | |
| 147 | 2-Br | $CF_2Cl$ | $COC_6H_4Cl$(4) | |
| 148 | 2-Br | $CF_2Cl$ | $COC_6H_4(CF_3)$(3) | |
| 149 | 2-Br | $CF_2Cl$ | $COC_6H_4(CF_3)$(2) | |
| 150 | 2-Br | $CF_2Cl$ | $COC_6H_4(OCF_3)$(4) | |
| 151 | 2-Br | $CF_2Cl$ | $COC_6H_4(NO_2)$(2) | |
| 152 | 2-Br | $CF_2Cl$ | $COC_6H_4Cl$(3) | |
| 153 | 2-Br | $CF_2Cl$ | $COC_6H_4(OCH_3)$(4) | |
| 154 | 2-Br | $CF_2Cl$ | $COC_6H_4(OCOCH_3)$(2) | |
| 155 | 2-Br | $CF_2Cl$ | $COC_6H_3(Cl_2)$(3,4) | |
| 156 | 2-Br | $CF_2Cl$ | $COC_6H_3(NO_2)(3)(CH_3)(4)$ | |
| 157 | 3-$NO_2$ | $C_2F_5$ | Nicotinoyl | |
| 158 | 3-$NO_2$ | $C_2F_5$ | Isonicotinoyl | |
| 159 | 3-$NO_2$ | $C_2F_5$ | 2,6-Dichlorisonicotinoyl | |
| 160 | 3-$NO_2$ | $C_2F_5$ | 3-Furoyl | |
| 161 | 3-$NO_2$ | $C_2F_5$ | Thienyl-2-carbonyl | |
| 162 | 3-$NO_2$ | $C_2F_3$ | 4-Methyl-1,2,3-thiadiazol-5-carbonyl | |
| 163 | 3-$NO_2$ | $C_2F_3$ | 3,5-Dimethyl-1,2-oxazolyl-2-carbonyl | |
| 164 | 3-$NO_2$ | $C_2F_3$ | 2,5-Dimethylfuroyl-2-carbonyl | |

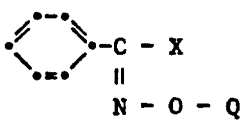

| Nr. | Phenylsubstitution | X | Q | phys. Daten |
|---|---|---|---|---|
| 165 | 3-$NO_2$ | $C_2F_3$ | 2-Methyl-4,5-dihydropyranyl-3-carbonyl | |
| 166 | 3-$NO_2$ | $C_2F_3$ | 3-Methyl-2,3-dihydro-1,4-thioxin-yl-2-carbonyl | |
| 167 | 3-$NO_2$ | $C_2F_3$ | 3-Methyl-2,3-dihydro-1-oxo-1,4-thioxinyl-2-carbonyl | |
| 168 | 3-$NO_2$ | $C_2F_3$ | 2,4-Dimethyl-1,4-thiazolyl-2-carbonyl | |
| 169 | 2,4-$Br_2$ | $CF_3$ | 1,4-Thiazolyl-2-carbonyl | |
| 170 | 2,4-$Br_2$ | $CF_3$ | 1,4-Dichlorpyrimidinyl-5-carbonyl | |
| 171 | 2,4-$Br_2$ | $CF_3$ | 3-Brom-2-furoyl | |
| 172 | 2,4-$Br_2$ | $CF_3$ | $CH_2C_6H_5$ | |
| 173 | 2-Cl, 4-Br | $CF_3$ | $CH_2C_6H_4Cl(4)$ | |
| 174 | 2-Cl, 4-Br | $CF_3$ | $CH_2C_6H_3Cl_2(2,4)$ | |
| 175 | 2-Cl, 4-Br | $CF_3$ | $CH_2C_6H_4Br(4)$ | |
| 176 | 2-Cl, 4-Br | $CF_3$ | $CH_2C_6H_4(CN)(4)$ | |
| 177 | 2-Cl, 4-Br | $CF_3$ | $CH_2C_6H_4(CH_3)(4)$ | |
| 178 | 2-Cl, 4-Br | $CF_3$ | $CH_2C_6H_4(C_3H_7i)(4)$ | |
| 179 | 2-Cl, 4-Br | $CF_3$ | $CH_2C_6H_4F(2)$ | |
| 180 | 2-Cl, 4-Br | $CF_3$ | $CH_2C_6H_3F_2(2,6)$ | |
| 181 | 2-Cl, 4-Br | $CF_3$ | $CH_2C_6H_4(NO_2)(4)$ | |
| 182 | 2-Cl, 4-Br | $CF_3$ | $CH_2C_6H_4(OCH_3)(3)$ | |
| 183 | 2-Cl, 4-Br | $CF_3$ | $C_2H_4C_6H_5$ | |
| 184 | 2-Cl, 4-Br | $CF_3$ | $CH_2C_6H_5(C_6H_5)(4)$ | |
| 185 | 4-$OCH_3$ | $C_3H_4F_3$ | $CONHCH_3$ | |
| 186 | 4-$OCH_3$ | $C_3H_4F_3$ | $CONH_2$ | |
| 187 | 4-$OCH_3$ | $C_3H_4F_3$ | $CONHC_2H_5$ | |
| 188 | 4-$OCH_3$ | $C_3H_4F_3$ | $CONHC_6H_5$ | |
| 189 | 4-$OCH_3$ | $C_3H_4F_3$ | $CONHC_6H_3(CH_3)_2(2,6)$ | |
| 190 | 4-$OCH_3$ | $C_3H_4F_3$ | CONH-Cyclohexyl | |
| 191 | 4-$OCH_3$ | $C_3H_4F_3$ | $CONHC_3H_7n$ | |
| 192 | 4-$OCH_3$ | $C_3H_4F_3$ | $CONHC_3H_7i$ | |
| 193 | 4-$OCH_3$ | $C_3H_4F_3$ | $CONHC_6H_4Cl(4)$ | |
| 194 | 4-$OCH_3$ | $C_3H_4F_3$ | $CONHC_4H_9t$ | |
| 195 | 4-$OCH_3$ | $C_3H_4F_3$ | $CONHC_6H_4(OC_2H_5)(4)$ | |
| 196 | 4-Cl | $CF_3$ | $CH_2COOCH_2CF_3$ | Sdp. 77-78°/0,06 mbar |
| 197 | 4-F | $CF_3$ | $CH_2COOCH_2CF_3$ | |
| 198 | 4-$CH_3$ | $CF_3$ | $CH_2COOCH_2CF_3$ | |
| 199 | 4-$OCF_3$ | $CF_3$ | $SO_2CH_3$ | |
| 200 | 4-$OCF_3$ | $CF_3$ | $SO_2CH_2Cl$ | |
| 201 | 4-$OCF_3$ | $CF_3$ | $SO_2C_6H_5$ | |
| 202 | 4-$OCF_3$ | $CF_3$ | $SO_2N(CH_3)_2$ | |
| 203 | 4-$OCF_3$ | $CF_3$ | $SO_2C_6H_4Cl(4)$ | |
| 204 | 4-$OCF_3$ | $CF_3$ | $SO_2C_6H_4(CH_3)(4)$ | |
| 205 | 4-$OCF_3$ | $CF_3$ | Piperidinosulfonyl | |
| 206 | 4-$OCF_3$ | $CF_3$ | Morpholinosulfonyl | |
| 207 | 4-$OCF_3$ | $CF_3$ | Piperidylsulfonyl | |
| 208 | 4-Cl | $CF_2Cl$ | Succinimidomethyl | |
| 209 | 4-Cl | $CF_3$ | 2,3-Dimethylmaleylimidomethyl | |
| 210 | — | $CF_3$ | Succinimidomethyl | |
| 211 | — | $CF_3$ | 2,3-Dimethylmaleylimidomethyl | |
| 212 | 4-Cl | $CF_3$ | Maleylimidomethyl | |
| 213 | — | $CF_3$ | Maleylimidomethyl | |
| 214 | 4-Cl | $CF_3$ | Phthalimidomethyl | |
| 215 | — | $CF_3$ | Phthalimidomethyl | |
| 216 | 4-Cl | $CF_3$ | 3,4,5,6-Tetrahydrophthalimido-methyl | |

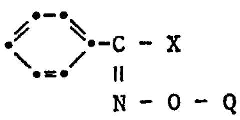

| Nr. | Phenylsubstitution | X | Q | phys. Daten |
|---|---|---|---|---|
| 217 | 4-Cl | $CF_3$ | 1,2,3,4-Tetrahydrophthalimido-methyl | |
| 218 | — | $CF_3$ | 3,4,5,6-Tetrahydrophthalimido-methyl | |
| 219 | — | $CF_3$ | 1,2,3,4-Tetrahydrophthalimido-methyl | |
| 220 | 4-Cl | $CF_3$ | $SO_2N(C_3H_7i)CH_2OCH_3$ | |
| 221 | 4-Cl | $CF_3$ | $SO_2N(CH_3)CH_2OCH_3$ | |
| 222 | 4-Cl | $CF_3$ | $SO_2N(C_2H_5)CH_2OCH_3$ | |
| 223 | 4-Cl | $CF_3$ | $SO_2CCl_3$ | |
| 224 | 4-Cl | $CHF_2$ | $CH_2CN$ | |
| 225 | 4-Cl | $CHF_2$ | $CH(CH_3)CN$ | |
| 226 | 4-Cl | $CHF_2$ | $CH_2CONH_2$ | |
| 227 | 4-Cl | $CHF_2$ | $CH(CH_3)CONH_2$ | |
| 228 | 4-Cl | $CF_3$ | 2,4,6-Trichlorpyridino | |
| 229 | 4-Cl | $CHF_2$ | 2,4,6-Trichlorpyridino | |
| 230 | 4-Cl | $CF_3$ | 2,4,6-Tribrompyridino | |
| 231 | 4-Cl | $CF_3$ | 3,5-Dichlorpyridino | |
| 232 | 4-Cl | $CF_3$ | 3,5-Dichlorpyridino | |
| 233 | 4-Cl | $CF_3$ | 5-Chlor-3-trifluormethylpyridino | |
| 234 | 4-Cl | $CHF_2$ | 5-Chlor-3-trifluormethylpyridino | |
| 235 | 4-$OCH_3$ | $CF_3$ | $CH_2CN$ | |
| 236 | 4-$OCH_3$ | $CF_3$ | $CH_2CONH_2$ | |
| 237 | — | $CF_3$ | 2,4,6-Trichlorpyridino | |
| 238 | — | $CF_3$ | 3,5-Dichlorpyridino | |
| 239 | — | $CF_3$ | 5-Chlor-3-trifluormethylpyridino | |
| 240 | — | $CF_3$ | $SO_2N(CH_3)CH_2OCH_3$ | |
| 241 | — | $CF_3$ | $SO_2N(C_2H_5)CH_2OCH_3$ | |
| 242 | — | $CF_3$ | $SO_2N(C_3H_7i)CH_2OCH_3$ | |
| 243 | — | $CF_3$ | $SO_2CCl_3$ | |
| 244 | — | $CHF_2$ | $CH_2CN$ | |
| 245 | — | $CHF_2$ | $CH_2OCH_3$ | |
| 246 | 4-Cl | $CF_3$ | $CH_2-C\equiv Cl$ | |
| 247 | — | $CF_3$ | $CH_2-C\equiv Cl$ | |
| 248 | — | $CF_3$ | $C_2H_4OH$ | |
| 249 | 4-Cl | $CF_3$ | $CH_2-COOCH_3$ | 78-80°/0,06 |
| 250 | — | $CF_3$ | $C_2H_4OH$ | |
| 251 | 4-Cl | $CF_3$ | $C_2H_4OH$ | |
| 252 | — | $CF_3$ | $C_2H_4OCOCH_3$ | |
| 253 | 4-Cl | $CF_3$ | $C_2H_4OCOCH_3$ | |
| 254 | — | $CF_3$ | $C_2H_4OCONHCH_3$ | |
| 255 | 4-Cl | $CF_3$ | $C_2H_4OCONHCH_3$ | |
| 256 | — | $CF_3$ | $C_2H_4OCH_2CH=CH_2$ | |
| 257 | 4-Cl | $CF_3$ | $C_2H_4OCH_2CH=CH_2$ | |
| 258 | 3-$CF_3$ | $CF_3$ | 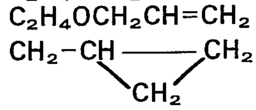 | Sdp. 57-57°/0,08 mbar |
| 259 | 3-$CF_3$ | $CF_3$ | $CH_2-CONH_2$ | wachsartig |
| 260 | — | $C_2F_5$ | $CH_2-CONH_2$ | Sdp. 90-100°/0,05 mbar |
| 261 | 3-$CF_3$ | $CF_3$ | $CH(CH_3)CONH_2$ | Sdp. 102-111°/0,08 mbar |
| 262 | 4-F | $CF_3$ | $CH_2C_6H_4F(2)$ | Sdp. 81-83°/0,03 mbar |
| 263 | 4-Cl | $CF_3$ | $CH_2C_6H_4F(2)$ | Sdp. 95-96°/0,08 mbar |
| 264 | 4-F | $CF_3$ | $CH_2C_6H_4F(4)$ | Sdp. 76-77°/0,04 mbar |
| 265 | 4-Cl | $CF_3$ | $CH_2C_6H_4F(4)$ | Sdp. 95-97°/0,05 mbar |
| 266 | 4-$CH_3$ | $CF_3$ | $CH_2CN$ | Sdp. 83-84°/0,1 mbar |
| 267 | 4-F | $CF_3$ | $CH_2CN$ | Sdp. 119-122°/12 mbar |
| 268 | 4-Cl | $CF_3$ | 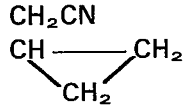 | |

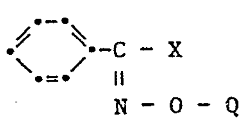

| Nr. | Phenylsubstitution | X | Q | phys. Daten |
|---|---|---|---|---|
| 269 | 4-Cl | $CF_3$ | $CH_2-CO-NHCH_3$ | |
| 270 | 4-Cl | $CF_3$ | $CH_2CON(CH_3)_2$ | |
| 271 | 4-Cl | $CF_3$ | $CH_2CONHC_2H_5$ | |
| 272 | 4-Cl | $CF_3$ | $CH_2COOC_2H_5$ | |
| 273 | 4-Cl | $CF_3$ | $CH(CH_3)COOC_2H_5$ | |
| 274 | 2-Cl | $CF_3$ | $CH_2CONH_2$ | Fp. = 70-73° |
| 275 | 2-Cl | $CF_3$ | $CH(CH_3)CONH_2$ | Kp. = 121-123°/0,12 |
| 276 | 2-Cl | $CF_3$ | $CH_2COOC_2H_5$ | |
| 277 | 2-Cl | $CF_3$ | $CH(CH_3)COOC_2H_5$ | |
| 278 | 4-Cl | $CF_3$ | $CH(CH_3)COOC_2H_5$ | |
| 279 | 4-Cl | $CF_3$ | $CH(CH_3)COOC_3H_7n$ | |
| 280 | 4-Cl | $CF_3$ | $CH(CH_3)COOCH_2CH=CH_2$ | |
| 281 | 4-Cl | $CF_3$ | $CH_2CSNH_2$ | |
| 282 | 4-Cl | $CF_3$ | $CH(CH_3)CSNH_2$ | |
| 283 | 4-$CH_3$ | $CF_3$ | $CH_2CONH_2$ | Fp. = 92-94° |
| 284 | 4-$CH_3$ | $CF_3$ | $CH(CH_2)CONH_2$ | Fp. = 76-77° |
| 285 | 3,4$(CH_3)_2$ | $CF_3$ | $CH_2CONH_2$ | |
| 286 | 3,4$(CH_3)_2$ | $CF_3$ | $CH(CH_3)CONH_2$ | |
| 287 | — | $C_2H_5$ | $CH_2CONH_2$ | |
| 288 | — | $C_2H_5$ | $CH(CH_3)CONH_2$ | |
| 289 | 4-$CH_3$ | $CF_3$ | $CH_2COOCH_3$ | |
| 290 | 4-$CH_3$ | $CF_3$ | $CH_2COOC_3H_7n$ | |
| 291 | 4-$CH_3$ | $CF_3$ | $CH(CH_3)COOC_2H_5$ | |
| 292 | 4-Cl | $CF_3$ | $CH_2COSC_2H_5$ | |
| 293 | 4-Cl | $CF_3$ | $CH_2COOC_2H_4OCH_3$ | |
| 294 | 4-Cl | $CF_3$ | $CH_2COOC_2H_4Cl$ | |
| 295 | 4-$OCH_3$ | $CF_3$ | $CH_2CONH_2$ | |
| 296 | 3-$NO_2$ | $CF_3$ | $CH_2CONH_2$ | |
| 297 | 4-Cl | $CHF_2$ | $CH_2CONH_2$ | |
| 298 | 4-Cl | $CHF_2$ | $CH(CH_3)CONH_2$ | |

*Beispiel 2:*

*Formulierungsbeispiele für Wirkstoffe der Formel I oder Mischungen dieser Wirkstoffe mit Herbiziden*

a) *Spritzpulver*

| | *a)* | *b)* | *c)* |
|---|---|---|---|
| Wirkstoff der Formel I oder Mischung mit Herbizid (%) | 20 | 60 | 0,5 |
| Na-Ligninsulfonat (%) | 5 | 5 | 5 |
| Na-Laurylsulfat (%) | 3 | — | — |
| Na-Diisobutylnaphthalinsulfonat (%) | — | 6 | 6 |
| Octylphenolpolyäthylenglykoläther (7-8 mol AeO) (%) | — | 2 | 2 |
| Hochdisperse Kieselsäure (%) | 5 | 27 | 27 |
| Kaolin (%) | 67 | — | — |
| Natriumchlorid (%) | — | — | 59,5 |

Der Wirkstoff wird mit den Zusatzstoffen gut vermischt und in einer geeigneten Mühle gut vermahlen. Man erhält Spritzpulver, die sich mit Wasser zu Suspension jeder gewünschten Konzentration verdünnen lassen.

b) *Emulsion-Konzentrat*

| | *a)* | *b)* |
|---|---|---|
| Wirkstoff der Formel I oder Mischung mit Herbizid | 10 | 1 |
| Octylphenolpolyäthylenglykoläther (4-5 mol AeO) (%) | 3 | 3 |
| Ca-Dodecylbenzolsulfonat | 3 | 3 |
| Ricinusölpolyglykoläther (36 mol AeO) (%) | 4 | 4 |
| Cyclohexanon (%) | 30 | 10 |
| Xylolgemisch (%) | 50 | 79 |

Aus diesem Konzentrat können durch Verdünnen mit Wasser Emulsionen jeder gewünschten Konzentration hergestellt werden.

c) *Stäubemittel*

| | *a)* | *b)* |
|---|---|---|
| Wirkstoff der Formel I oder Mischung mit Herbizid (%) | 0,1 | 1 |
| Talkum (%) | 99,9 | — |
| Kaolin (%) | — | 99 |

Man erhält anwendungsfertige Stäubemittel, indem der Wirkstoff mit dem Träger vermischt und auf einer geeigneten Mühle vermahlen wird.

d) *Extruder Granulat*

| | a) | b) |
|---|---|---|
| Wirkstoff der Formel I oder Mischung mit Herbizid (%) | 10 | 1 |
| Na-Ligninsulfonat (%) | 2 | 2 |
| Carboxymethylcellulose (%) | 1 | 1 |
| Kaolin (%) | 87 | 96 |

Der Wirkstoff wird mit den Zusatzstoffen vermischt, vermahlen und mit Wasser angefeuchtet. Dieses Gemisch wird extrudiert und anschliessend im Lufstrom getrocknet.

e) *Umhüllungs-Granulat*

| | |
|---|---|
| Wirkstoff der Formel I oder Mischung mit Herbizid (%) | 3 |
| Polyäthylenglykol (MG 200) | 3 |
| Kaolin | 94 |

Der fein gemahlene Wirkstoff wird in einem Mischer auf das mit Polyäthylenglykol angefeuchtete Kaolin gleichmässig aufgetragen. Auf diese Weise erhält man staubfreie Umhüllungs-Granulate.

f) *Suspensions-Konzentrat*

| | a) | b) |
|---|---|---|
| Wirkstoff der Formel I oder Mischung mit Herbizid (%) | 40 | 5 |
| Äthylenglykol (%) | 10 | 10 |
| Nonylphenolpolyäthylenglykoläther (15 mol AeO) (%) | 6 | 1 |
| Na-Ligninsulfonat (%) | 10 | 5 |
| Carboxymethylcellulose (%) | 1 | 1 |
| 37%ige wässerige Formaldehyd-Lösung | 0,2 | 0,2 |
| Silikonöl in Form einer 75%igen wässerigen Emulsion (%) | 0,8 | 0,8 |
| Wasser (%) | 32 | 77 |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

g) *Salzlösung*

| | |
|---|---|
| Wirkstoff der Formel I oder Mischung mit Herbizid (%) | 5 |
| Isopropylamin (%) | 1 |
| Octylphenolpoyäthylenglykoläther (78 mol AeO) (%) | 3 |
| Wasser (%) | 91 |

*Biologische Beispiele*

Die Fähigkeit der Verbindungen der Formel I, Kulturpflanzen vor der phytotoxischen Wirkung starker Herbizide zu schützen, kann aus den folgenden Beispielen ersehen werden. In den Versuchsbeschreibungen werden die Verbindungen der Formel I als Antidote (Gegenmittel) bezeichnet.

*Beispiel 3:*

Versuch mit Antidote und Herbizide in Sorghum. Applikation von Herbizide und Antidote als Tankmischung im Vorauflauf.

Plastikcontainer von 25 × 17 $cm^2$ Bodenfläche und 12 cm Höhe werden mit sandiger Lehmerde gefüllt und mit Sorghumsamen der Sorte Funk 522 besät. Dann werden die Samen mit einer dünnen Schicht Erde bedeckt. Darauf sprüht man eine verdünnte wässerige Lösung, welche das Herbizid und das Antidote in der gewünschten Mischung enthält. Der Zustand der Pflanzen wird nach 30 d beurteilt. Die Schutzwirkung wird in Prozent ausgedrückt und gibt an um wieviel das Antidote oder Gegenmittel die phytotoxische Wirkung des Herbizides zu verringern vermag. Eine signifikante Wirkung wird erreicht, wenn es gelingt, die Phytotoxizität aus dem Bereich der schweren oder mittleren Schäden in dasjenige der leichten, reversiblen Schäden zurückzudrängen oder ganz aufzuheben.

Die Resultate sind wie folgt:

*Herbizid:*

N-Chloracetyl-N-(2-methoxy-1-methyläthyl)-2-äthyl-6-methylanilin (Metolachlor)

| Antidote Nr. | kg/ha | Herbizid (kg/ha) | relative Schutzwirkung in % |
|---|---|---|---|
| 4 | 1,5 | 1,5 | 25 |
| 5 | 1,5 | 1,5 | 13 |
| 8 | 1,5 | 1,5 | 25 |
| 14 | 1,5 | 1,5 | 63 |
| 71 | 1,5 | 1,5 | 13 |
| 81 | 1,5 | 1,5 | 38 |
| 83 | 1,5 | 1,5 | 25 |
| 84 | 1,5 | 1,5 | 63 |
| 85 | 1,5 | 1,5 | 13 |
| 259 | 1,5 | 1,5 | 50 |
| 261 | 1,5 | 1,5 | 38 |
| 264 | 1,5 | 1,5 | 38 |
| 267 | 1,5 | 1,5 | 25 |

*Beispiel 4:*

Versuch mit Antidote und Herbizid in Sorghum im Vorauflaufverfahren. Applikation der Antidote durch Samenbeizung.

Sorghum der Sorte Funk G 522 werden mit dem Antidote in einem Glaskolben zusammengegeben. Samen und Produkt werden durch Schütteln und Rotation des Glaskolbens gut durchgemischt. Anschliessend wird der so gebeizte Samen in mit sandiger Erde gefüllte Blumentöpfe (oberer Durchmesser 11 cm) eingesät. Der Samen wird mit einer dünnen Erdschicht bedeckt. Darauf wird nun ein wässerige Herbizidemulsion in der gewünschten Applikationsmenge gesprüht. Der Zustand der Pflanzen wird 8 d nach der Herbizidbehandlung beurteilt und die relative Schutzwirkung des Safeners wird in Prozent ausgedrückt. Die Resultate sind wie folgt:

*Herbizid:* N-Chloracetyl-N-(2-methoxy-1-methyläthyl)-2-äthyl-6-methylanilin (Metolachlor)

| Antidote Nr. | g/kg Saatgut | Herbizid (kg/ha) | relative Schutzwirkung in % |
|---|---|---|---|
| 5 | 2 | 4 | 88 |
| 5 | 1 | 4 | 50 |
| 5 | 0,5 | 4 | 25 |
| 5 | 2 | 2 | 63 |
| 5 | 1 | 2 | 50 |
| 5 | 0,5 | 2 | 50 |
| 5 | 2 | 1 | 75 |
| 5 | 1 | 1 | 38 |
| 5 | 0,5 | 1 | 38 |
| 13 | 2 | 4 | 50 |
| 13 | 1 | 4 | 50 |
| 13 | 0,5 | 4 | 25 |
| 13 | 2 | 2 | 75 |
| 13 | 1 | 2 | 63 |
| 13 | 0,5 | 2 | 25 |
| 13 | 2 | 1 | 75 |
| 13 | 1 | 1 | 63 |
| 13 | 0,5 | 1 | 38 |
| 84 | 2 | 4 | 38 |
| 84 | 1 | 4 | 50 |
| 84 | 0,5 | 4 | 50 |
| 84 | 2 | 2 | 38 |
| 84 | 1 | 2 | 50 |
| 84 | 0,5 | 2 | 50 |
| 84 | 2 | 1 | 38 |
| 84 | 1 | 1 | 50 |
| 84 | 1 | 1 | 50 |

*Beispiel 5:*

Versuche mit Antidote und Herbizid in Soja. Applikation von Antidote und Herbizid als Tankmischung im Vorauflauf.

Blumentöpfe (oberer Durchmesser 6 cm) werden mit sandiger Lehmerde gefüllt und Sojasamen der Sorte Hark werden eingesät. Nach dem Bedekken der Samen wird die als Antidote zu prüfende Substanz zusammen mit dem Herbizid in verdünnter Lösung als Tankmischung auf die Bodenoberfläche versprüht. 21 d nach der Herbizidapplikation wird die Schutzwirkung des Antidots in % bonitiert. Das Resultat ist wie folgt:

*Herbizid:* 4-Amino-3-methylthio-6-tert.-butyl-1,2,4-triazin-5-on (Metribuzin)

| Antidote Nr. | kg/ha | Herbizid (kg/ha) | relative Schutzwirkung in % |
|---|---|---|---|
| 1 | 1,5 | 0,75 | 50 |

*Beispiel 6:*

Versuch mit Antidote und Herbizid in Weizen. Applikation von Antidote und Herbizid als Tankmischung im Vorauflauf.

Weizensamen der Sorte Farnese werden in Plastiktöpfe (oberer Durchmesser 11 cm), die 0,5 l Gartenerde enthalten, im Gewächshaus ausgesät. Nach dem Bedecken der Samen wird die als Antidote zu prüfende Substanz zusammen mit dem Herbizid als Tankmischung appliziert. 24 d nach der Applikation wird die Schutzwirkung des Antidots in Prozent bonitiert.

Die Resultate sind wie folgt:

*Herbizid:* α-[4-(3,5-Dichlorpyridyl-2-oxy)phenoxy]propionsäurepropinylester

| Antidote Nr. | kg/ha | Herbizid (kg/ha) | relative Schutzwirkung in % |
|---|---|---|---|
| 5 | 1,5 | 0,75 | 38 |
| 8 | 1,5 | 0,75 | 25 |
| 14 | 1,5 | 0,75 | 38 |
| 72 | 1,5 | 0,75 | 25 |
| 79 | 1,5 | 0,75 | 38 |
| 81 | 1,5 | 0,75 | 25 |
| 84 | 1,5 | 0,75 | 38 |
| 85 | 1,5 | 0,75 | 38 |

*Beispiel 7:*

Versuch mit Antidote und Herbizid in Mais. Applikation des Gegenmittels durch Samenbeizung.

Maissamen der Sorte LG 5 werden mit der als Safener zu prüfenden Substanz in einen Glasbehälter gemischt. Samen und Produkt werden durch Schütteln und Rotation gut zusammengemischt. Plastiktöpfe (oberer ⌀ 11 cm) werden mit Erde gefüllt und die gebeizten Samen werden eingesät. Nach dem Bedecken der Samen wird z. T. das Herbizid in beträchtlicher Überdosierung im Vorauflauf appliziert. 18 d nach der Herbizidapplikation wird die Schutzwirkung des Safeners in Prozent bonitiert. Als Referenzen dienen dabei die mit Herbizid allein behandelten Pflanzen sowie die vollständig unbehandelte Kontrolle. Die Resultate sind wie folgt:

*Herbizid:* N-Chloracetyl-N-(2-methoxy-1-methyläthyl)-2-äthyl-6-methylanilin (Metolachlor)

| Antidote Nr. | g/kg Saatgut | Herbizid (kg/ha) | relative Schutzwirkung in % |
|---|---|---|---|
| 84 | 2 | 6 | 75 |
| 84 | 1 | 6 | 75 |
| 84 | 1,5 | 6 | 75 |

*Herbizid:* N,N-Di-n-propyl-5-äthyl-thiocarbamat (EPTC)

| Antidote Nr. | g/kg Saatgut | Herbizid (kg/ha) | relative Schutzwirkung in % |
|---|---|---|---|
| 84 | 2 | 6 | 50 |
| 84 | 1 | 6 | 63 |
| 84 | 0,5 | 6 | 38 |
| 84 | 2 | 4 | 63 |
| 84 | 1 | 4 | 63 |
| 84 | 0,5 | 4 | 38 |

*Herbizid:* N-Isopentyl-3,4-dimethyl-2,6-dinitroanilin (Penoxalin)

| Antidote Nr. | g/kg Saatgut | Herbizid (kg/ha) | relative Schutzwirkung in % |
|---|---|---|---|
| 84 | 2 | 4 | 50 |
| 84 | 1 | 4 | 50 |
| 84 | 0,5 | 4 | 25 |
| 84 | 2 | 2 | 25 |
| 84 | 2 | 2 | 25 |
| 84 | 0,5 | 2 | 38 |

*Beispiel 8:*

Versuch mit Antidote und Herbizid in Mais. Applikation von Antidote und Herbizid als Tankmischung im Vorauflauf.

Plastikcontainer (25 cm lang × 17 cm breit × 12 cm hoch) wurden mit sandiger Lehmerde gefüllt und Maissamen der Sorte LG 5 eingesät. Nach dem Bedecken der Samen wurde die als Safener zu prüfenden Substanz zusammen mit dem Herbizid (in Überdosierung) in verdünnter Lösung als Tankmischung auf die Bodenoberfläche gesprüht. 21 d nach der Applikation wurde die Schutzwirkung des Safeners in Prozent bonitiert. Als Referenzen dienen dabei die mit dem Herbizid allein behandelten Pflanzen sowie die vollständig unbehandelte Kontrolle. Die Resultate sind wie folgt:

*Herbizid:* N-Chloracetyl-N-(2-methoxy-1-methyläthyl)-2-äthyl-6-methylanilin (Metolachlor)

| Antidote Nr. | kg/ha | Herbizid (kg/ha) | relative Schutzwirkung in % |
|---|---|---|---|
| 84 | 3 | 6 | 50 |
| 84 | 1,5 | 6 | 38 |
| 84 | 0,75 | 6 | 25 |

*Beispiel 9:*

Versuch mit Antidote und Herbizid in trocken gesätem Reis. Applikation von Herbizid und Antidote als Tankmischung im Vorauflauf.

Reissamen werden in Container (47 cm lang, 29 cm breit und 24 cm hoch) eingesät, bedeckt und leicht festgedrückt. Dann wird die als Antidote zu prüfende Substanz als verdünnte Lösung zusammen mit dem Herbizid als Tankmischung versprüht. 24 d nach der Saat wird die Schutzwirkung des Antidots in Prozent bonitiert. Als Referenzen dienen dabei die mit Herbizid allein behandelten Pflanzen sowie die vollständig unbehandelte Kontrolle. Das Resultat ist wie folgt:

*Herbizid:* N-Chloracetyl-N-(2-methoxy-1-methylalkyl)-2-äthyl-6-methylanilin (Metolachlor)

| Antidote Nr. | kg/ha | Herbizid (kg/ha) | relative Schutzwirkung in % |
|---|---|---|---|
| 260 | 1,5 | 0,25 | 25 |

*Beispiel 10:*

Versuch mit Antidote und Herbizid in trocken gesätem und nachher überflutetem Reis. Applikation von Herbizid und Antidote als Tankmischung im Vorauflauf.

Reissamen der Sorte IR-36 werden in Container (47 cm lang, 29 cm breit und 24 cm hoch) eingesät, bedeckt und leicht festgedrückt. Dann wird die als Antidote zu prüfende Substanz zusammen mit dem Herbizid als Tankmischung versprüht. Etwa 20 d nach der Saat (3-Blattstadium der Reispflanzen) wird die Bodenoberfläche mit 4 cm Wasser Höhe beschichtet. 30 d nach der Applikation wird die Schutzwirkung des Antidots in Prozent bonitiert. Als Referenzen dienen dabei die mit Herbizid allein behandelten Pflanzen sowie die vollständig unbehandelte Kontrolle.

Das Resultat ist wie folgt:

*Herbizid:* N-Chloracetyl-N-(2-methoxy-1-methyläthyl)-2-äthyl-6-methylanilin (Metolachlor)

| Antidote Nr. | kg/ha | Herbizid (kg/ha) | relative Schutzwirkung in % |
|---|---|---|---|
| 260 | 1,5 | 0,25 | 25 |

*Beispiel 11:*

Versuch mit Antidote und Herbizid in im Wasser gesätem Reis. Applikation des Gegenmittels während der Samenquellung.

Reissamen werden während 48 h mit Lösungen der als Safener zu prüfenden Substanz von 10, 100 und 1000 ppm getränkt. Anschliessend werden die Samen etwa 2 h trocknen gelassen, bis sie nicht mehr kleben. Plastikcontainer (25 cm lang, 17 cm breit und 12 cm hoch) werden bis 2 cm unter dem Rand mit sandigem Lehm gefüllt. Die vorgequollenen Samen werden auf der Bodenoberfläche des Containers gesät und nur ganz schwach gedeckt. Die Erde wird in einem feuchten (nicht sumpfigen) Zustand gehalten. Dann wird das Herbizid in verdünnter Lösung auf die Bodenoberfläche versprüht. Der Wasserstand wird entsprechend dem Wachstum sukzessiv erhöht. 18 d nach der Herbizidapplikation wird die Schutzwirkung des Safeners in Prozent bonitiert. Als Referenzen dienen dabei die mit dem Herbizid allein behandelten Pflanzen sowie die vollständig unbehandelte Kontrolle. Die Resultate sind wie folgt:

*Herbizid:* N-Chloracetyl-N-(2-n-Propyloxyäthyl)-2,6-diäthylanilin (Pretilachlor)

| Antidote Nr. | ppm | Herbizid (kg/ha) | relative Schutzwirkung in % |
|---|---|---|---|
| 18 | 100 | 0,25 | 25 |
| 260 | 100 | 0,25 | 50 |

## Patentansprüche

1. Oximäther der Formel I

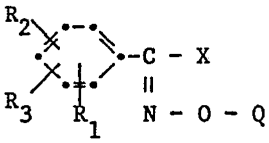 (I)

in welche $R_1$ Wasserstoff, Halogen, niederes Alkyl, niederes Halogenalkyl, niederes Alkoxy, niederes Halogenalkoxy, niederes Alkylthio, niederes Halogenalkylthio, niederes Alkylsulfinyl, niederes Alkylsulfonyl, niederes Halogenalkylsulfinyl, niederes Halogenalkylsulfonyl oder Nitro,

$R_2$ und $R_3$ je Wasserstoff, Halogen, niederes Alkyl, niederes Alkoxy oder niederes Halogenalkyl,

Q ein niederes Alkyl, des geradkettig oder verzweigt ist, oder durch Heteroatome unterbrochen und

a) durch Halogenatome, die Cyan- oder Oxygruppe substituiert ist oder

b) durch eine Phenoxy- oder Thiophenoxygruppe substituiert ist oder

– ein niederes Alkenyl oder Halogenalkenyl,
– ein niederes Alkinyl oder Halogenalkinyl,
– einen 3-7gliedrigen Cycloalkylrest,
– einen niederen Alkancarbonsäureesterrest,
– einen niederen Alkenylcarbonsäureesterrest,
– einen niederen Alkancarbonsäureamid- oder Alkancarbonsäurehydrazidrest,
– einen niederen Alkancarbonsäurethioamidrest,
– einen niederen Alkancarbonsäurethiolesterrest,
– einen niederen Alkancarbonsäurerest oder ein Salz davon,
– einen aliphatischen oder araliphatischen Acylrest,
– einen halogensubstituierten aliphatischen Acylrest,
– einen durch Alkoxy oder Phenoxy substituierten aliphatischen Acylrest,
– einen cycloaliphatischen, aromatischen oder heterocyclischen Acylrest,
– einen substituierten oder unsubstituierten Arylrest,
– einen aliphatischen, cycloaliphatischen oder aromatischen Kohlensäurerest,
– einen aliphatischen, cycloaliphatischen oder aromatischen Thiokohlensäurerest,
– den Carbamoylrest, einen Cyanoalkylcarbamoylrest oder falls X nicht die Trifluormethylgruppe darstellt, auch einen Alkylcarbamoylrest,
– einen Alkyl- oder Arylsulfonylrest,
– einen Alkoxyalkylsulfonylamido oder einen Sulfonylamidorest,
– einen halogensubstituierten Alkyl- oder Arylsulfonylrest,
— einen cyclischen Acylamidomethylrest

worin 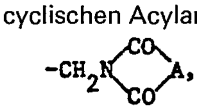, worin

A eine Alkylenkette, eine halogenierte Alkylenkette, eine Alkenylkette oder eine halogenierte Alkenylenkette bedeutet,

– einen Phthalimidomethylrest der teilweise oder ganz gesättigt sein kann und im Phenylkern gemäss $R_1$, $R_2$ und $R_3$ substituiert sein kann,
– eines 5-6gliedrigen gesättigten, teilweise gesättigten oder aromatischen heterocyclus, der 1-3 Stickstoff, Sauerstoff und/oder Schwefelatome enthalten kann und der gemäss $R_1$, $R_2$ und $R_3$ substituiert sein kann, und

X einen fluorierten $C_1$-$C_3$-Alkylrest, der auch Chlor enthalten kann, bedeuten.

2. Oximäther gemäss Anspruch 1 der Formel Ia

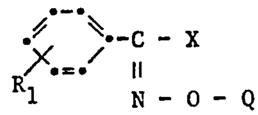 (Ia)

worin $R_1$ Q und X die im Anspruch 1 gegebene Bedeutung haben.

3. Oximäther gemäss Anspruch 1 der Formel (Ib)

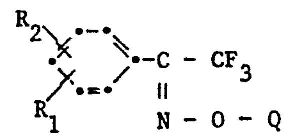 (Ib)

worin $R_1$ und Q die im Anspruch 1 gegebene Bedeutung haben.

4. Oximäther gemäss Anspruch 1 der Formel (Ic)

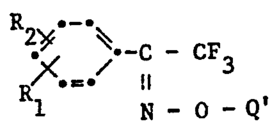 (Ic)

worin Q′ $C_1$-$C_4$-Alkyl, $C_1$-$C_4$-Cyanoalkyl, Carbamoyl $CONH_2$ bedeutet und $R_1$ und $R_2$ die im Anspruch 1 gegebene Bedeutung hat.

5. Oximäther gemäss Anspruch 1 der Formel (Ib)

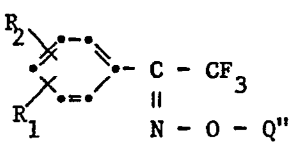 (Id)

worin Q″ $C_1$-$C_4$-Alkyl oder $C_1$-$C_4$-Cyanoalkyl bedeutet und $R_1$ die im Anspruch 1 gegebene Bedeutung hat.

6. Oximäther gemäss Anspruch 1 der Formel Ie

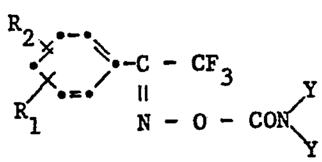 (Ie)

worin Y Wasserstoff bedeutet und $R_1$ und $R_2$ die im Anspruch 1 gegebene Bedeutung hat.

7. Oximäther gemäss Anspruch 1 der Formel (If)

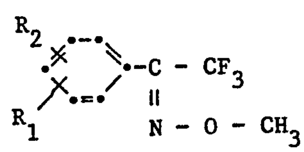 (If)

worin $R_1$ die im Anspruch 1 gegebene Bedeutung hat.

8. Oximäther gemäss Anspruch 1 der Formel (Ig)

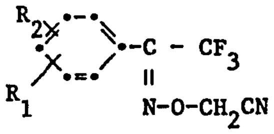

(Ig)

worin $R_1$ und $R_2$ die im Anspruch 1 gegebene Bedeutung hat.

9. Oximäther gemäss Anspruch 1 der Formel (Ih)

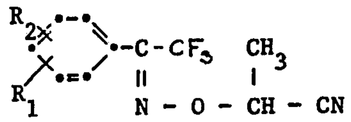

(Ih)

worin $R_1$ und $R_2$ die im Anspruch 1 gegebene Bedeutung hat.

10. Oximäther gemäss Anspruch 1 der Formel (Ii)

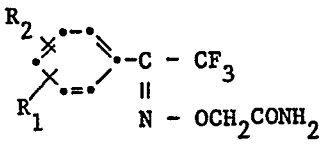

(Ii)

worin $R_1$ und $R_2$ die im Anspruch 1 gegebene Bedeutung hat.

11. Oximäther gemäss Anspruch 1 der Formel (Ij)

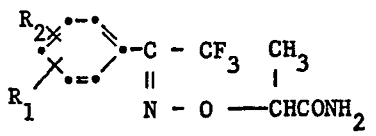

(Ij)

worin $R_1$ und $R_2$ die im Anspruch 1 gegebene Bedeutung hat.

12. Oximäther gemäss Anspruch 1, worin $R_1$ Wasserstoff, Halogen, Methyl, Methoxy, Trifluormethyl oder Nitro, $R_2$ Wasserstoff, Halogen oder Methyl und $R_3$ Wasserstoff bedeuten.

13. 1-Phenyl-1-methoximino-2,2,2-trifluoräthan gemäss Formel (I) der Formel

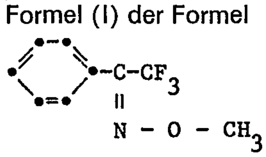

14. 1-(3-Trifluormethylphenyl)-1-cyclopropylmethoximino-2,2,2-trifluoräthan gemäss Anspruch 1 der Formel

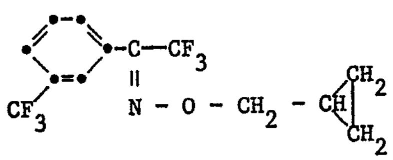

15. 1-(4-Chlorphenyl)-1-n-propoximino-2,2,2-trifluoräthan gemäss Anspruch 1 der Formel

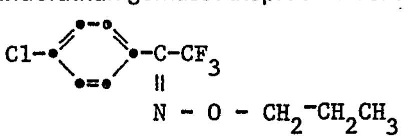

16. 1-(4-Chlorphenyl)-1-allyloximino-2,2,2-trifluoräthan gemäss Anspruch 1 der Formel

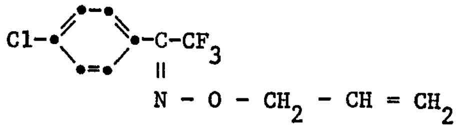

17. 1-Phenyl-1-carbamoylmethoximino-2,2,2-trifluoräthan gemäss Anspruch 1 der Formel

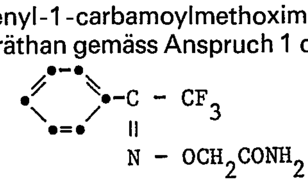

18. 1-(4-Fluorphenyl)-1-carbamoylmethoximino-2,2,2-trifluoräthan gemäss Anspruch 1 der Formel

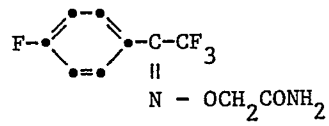

19. 1-(4-Chlorphenyl)-1-carbamoylmethoximino-2,2,2-trifluoräthan gemäss Anspruch 1 der Formel

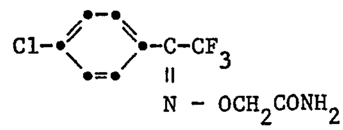

20. 1-(3-Trifluormethylphenyl)-1-carbamoylmethoximino-2,2,2-trifluoräthan gemäss Anspruch 1 der Formel

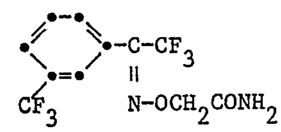

21. 1-Phenyl-1-carbamoylmethoximino-2,2,3,3,3-pentafluorpropan gemäss Anspruch 1 der Formel

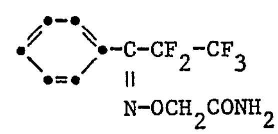

22. 1-Phenyl-1-carbamoyläth-1'-oximino-2,2,2-trifluoräthan gemäss Anspruch 1 der Formel

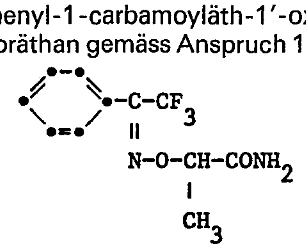

23. 1-(4-Chlorphenyl)-1-carbamoyläth-1'-oximino-2,2,2-trifluoräthan gemäss Anspruch 1 der Formel

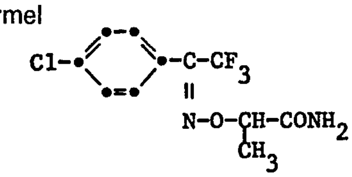

24. 1-(3-Trifluormethylphenyl)-1-carbamoyläth-1'-oximino-2,2,2-trifluoräthan gemäss Anspruch 1 der Formel

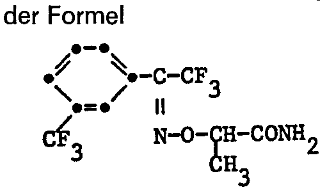

25. 1-(3-Chlorphenyl)-1-isopropyloxycarbonylmethoximino-2,2,2-trifluoräthan gemäss Anspruch 1 der Formel

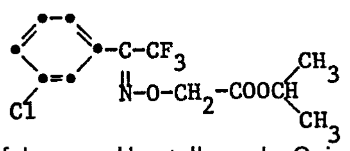

26. Verfahren zur Herstellung der Oximäther der Formel (I) Anspruch 1, dadurch gekennzeichnet, dass man ein Salz eines Oxims der Formel (II)

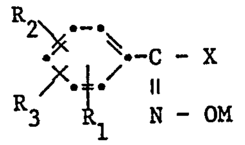

(II)

in welcher M ein Alkali- oder Erdalkalimetallkation darstellt und $R_1$, $R_2$, $R_3$ und X die im Anspruch 1 gegebene Bedeutung haben, mit einem reaktionsfähigen Ester der Formel (III) umsetzt

$$Y-Q \qquad (III)$$

worin Q die Anspruch 1 gegebene Bedeutung hat und Y einen organischen oder anorganischen Säurerest darstellt.

27. Verfahren nach Anspruch 17, dadurch gekennzeichnet, dass man als Salz eines Oxims der Formel (II) das Natrium- oder Kaliumsalz verwendet.

28. Verfahren nach Anspruch 17, dadurch gekennzeichnet, dass B ein Halogenatom oder einen Sulfonsäurerest darstellt.

29. Verfahren nach Anspruch 17, dadurch gekennzeichnet, dass man die Umsetzung des Oxims der Formel (II) mit dem reaktionsfähigen Ester der Formel (III) in einem polaren Lösungsmittel durchführt.

30. Verfahren gemäss Anspruch 17, dadurch gekennzeichnet, dass man das Hydroximin der Formel (II) oder ein Salz davon mit einem Sulfonylester der Formel (IV) umsetzt

$$Z-SO_3-Q \qquad (IV)$$

worin Z einen niederen Alkylrest oder einen durch Niederalkyl oder Halogen substituierten Phenylrest bedeutet und Q die im Anspruch 1 gegebene Bedeutung hat.

31. Mittel zum Schützen von Kulturpflanzen vor Schädigung durch Herbizide, dadurch gekennzeichnet, dass es neben inerten Träger- und Zusatztoffen als wirksame Komponente einen Oximäther der Formel (I) gemäss Anspruch 1 enthält.

32. Mittel nach Anspruch 32, gekennzeichnet durch einen Gehalt an

a) einem herbizid wirksamen Halogenacetanilid oder einem herbizid wirksamen Thiolcarbamat, und

b) einem Oximäther der Formel (I) gemäss Anspruch 1 als Gegenmittel.

33. Mittel nach Anspruch 32, gekennzeichnet durch einen Gehalt an

a) einem Halogenacetanilid der Formel (VII)

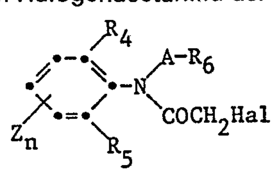

(VII)

in welcher Hal Halogen, insbesondere Fluor oder Brom, $R_4$ und $R_5$ unabhängig voneinander je Wasserstoff, Halogen, niederes Alkyl, Alkoxy, Alkylthio, Halogenalkyl, Alkoxyalkyl oder Alkylthioalkyl, Z Wasserstoff, Halogen, niederes Alkyl, Alkoxy, Alkylthio, Halogenalkyl, Alkoxyalkyl oder Alkylthioalkyl, wobei die Reste Z vorzugsweise in 3-Stellung in bezug auf das Stickstoffatom stehen, n 0 bis 3, A Alkylen, insbesondere Methylen, 1,1- und 1,2-Äthylen, wobei 1,2-Äthylen durch 1-2 niedere Alkylgruppen substituiert sein kann, und $R_6$ niederes Alkoxy, Hydroxycarbonyl, Alkoxycarbonyl, Carbamoyl, N-Alkylcarbamoyl, N,N-Dialkylcarbamoyl, Cyano, einen ggf. substituierten Stickstoffhaltigen heterocyclischen Rest, Alkanoyl, ggf. substituiertes Benzyl, ggf. substituiertes 1,3,4-Oxadiazol-2-yl, 1,3,4-Thiadiazol-2-yl, 1,3,4-Triazol-3-yl oder 1,3,4-Triazol-1-yl bedeutet, und

b) einem Oximäther der Formel (I) gemäss Anspruch 1 als Gegenmittel.

34. Mittel nach Anspruch 32, gekennzeichnet durch einen Gehalt an

a) einem Thiolcarbamat der Formeln (VIII) und (IX)

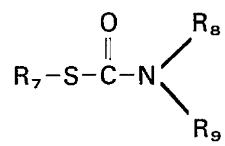

(VIII)

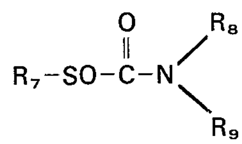

(IX)

in welcher $R_7$ niederes Alkyl, Alkenyl, Chlorallyl, Dichlorallyl, Trichlorallyl, Benzyl, oder 4-Chlorbenzyl, $R_8$ $C_2$-$C_4$-Alkyl und $R_9$ $C_2$-$C_4$-Alkyl oder Cyclohexyl bedeutet, wobei die Reste $R_8$ und $R_9$ zusammen mit dem Stickstoffatom, an das sie gebunden sind, einen Hexahydro-1H-azepin, Dekahydrochinolin- oder 2-Methyldekahydrochinolin-Ring bilden können, und

b) einem Oximäther der Formel (I) gemäss Anspruch 1 als Gegenmittel.

35. Verwendung der Oximäther der Formel (I) gemäss Anspruch 1 zum Schützen von Kulturpflanzen gegen die schädigende Wirkung von Herbiziden.

36. Verfahren zum Schützen von Kulturpflanzen vor Schäden, die bei der Applikation von Herbiziden auftreten, dadurch gekennzeichnet, dass man

a) die Anbaufläche für die Pflanze vor oder während der Applikation des Herbizids oder

b) den Samen oder die Stecklinge der Pflanzen oder die Pflanze selbst mit einer wirksamen Menge eines Oximäthers der Formel (I) gemäss Anspruch 1 behandelt.

## Claims

1. An oxime ether of the Formula (I)

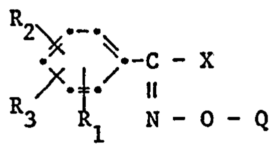

(I)

wherein

$R_1$ is hydrogen, halogen, lower alkyl, lower haloalkyl, lower alkoxy, lower haloalkoxy, lower alkylthio, lower haloalkylthio, lower alkylsulfinyl, lower alkylsulfonyl, lower haloalkylsulfonyl, lower haloalkylsulfonyl or nitro,

$R_2$ and $R_3$ are each hydrogen, halogen, lower alkyl, lower alkoxy or lower haloalkyl,

Q is lower alkyl which is straight chain or branched or interrupted by hetero atoms and is substituted

a) by halogen atoms or by the cyano or oxy group, or

b) by a phenoxy or thiophenoxy group, or is also lower alkenyl or haloalkenyl; lower alkynyl or haloalkynyl; a 3- to 7-membered cycloalkyl radical; a lower alkanoyl radical; a lower alkenoyl radical; a lower alkylcarbamoyl or hydrazidocarbonyl radical; a lower alkylthiocarbamoyl radical; a lower alkylthiocarboxy radical; a lower alkanoyl radical or a salt thereof; an aliphatic or araliphatic acyl radical; a halogen-substituted aliphatic acyl radical; an aliphatic acyl radical which is substituted by alkoxy or phenoxy; a cycloaliphatic, aromatic or heterocyclic acyl radical; a substituted or unsubstituted aryl radical; an aliphatic, cycloaliphatic or aromatic carbonic acid radical; an aliphatic, cycloaliphatic or aromatic thiocarbonic acid radical; the carbamoyl radical, a cyanoalkylcarbamoyl radical or, if X is not the trifluoromethyl group, is also an alkylcarbamoyl radical; an alkyl or arylsulfonyl radical; an alkoxyalkylsulfamoyl or sulfamoyl radical; a halogen-substituted alkylsulfonyl or arylsulfonyl radical; a cyclic acylamidomethyl radical

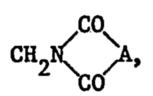

wherein A is an alkylene chain, a halogenated alkylene chain, an alkenyl chain or a halogenated alkenyl chain; a phthalimidomethyl radical which may be partially or completely saturated and may be substituted in the phenyl nucleus as indicated for $R_1$, $R_2$ and $R_3$; a 5- or 6-membered saturated, partially saturated or aromatic heterocyclic ring which may contain 1 to 3 nitrogen, oxygen and/or sulfur atoms and may be substituted as indicated for $R_1$, $R_2$ and $R_3$; and

X is a fluorinated $C_1$-$C_3$-alkyl radical which may also contain chlorine.

2. An oxime ether according to Claim 1 of the Formula (Ia)

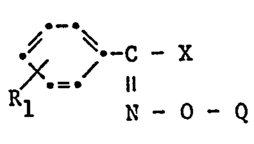

(Ia)

wherein $R_1$, Q and X are as defined in Claim 1.

3. An oxime ether according to Claim 1 of the Formula (Ib)

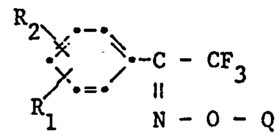

(Ib)

wherein $R_1$ and Q are as defined in Claim 1.

4. An oxime ether according to Claim 1 of the Formula (Ic)

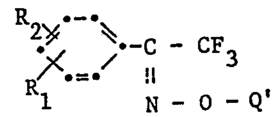

(Ic)

wherein Q' is $C_1$-$C_4$-alkyl, $C_1$-$C_4$-cyanoalkyl, carbamoyl, $CONH_2$, and $R_1$ and $R_2$ are as defined in Claim 1.

5. An oxime ether according to Formula (Id)

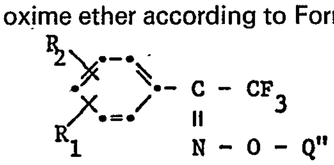

(Id)

wherein Q'' is $C_1$-$C_4$-alkyl or $C_1$-$C_4$-cyanoalkyl and $R_1$ is as defined in Claim 1.

6. An oxime ether according to Claim 1 to the Formula (Ie)

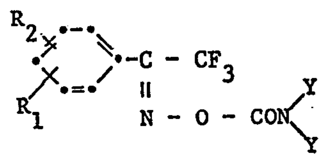

(Ie)

wherein Y is hydrogen and $R_1$ and $R_2$ are as defined in Claim 1.

7. An oxime ether according to Claim 1 of the Formula (If)

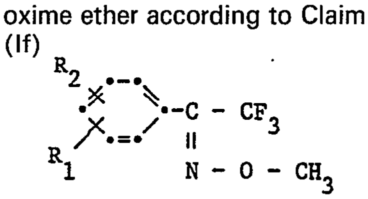

(If)

wherein $R_1$ is as defined in Claim 1.

8. An oxime ether according to Claim 1 of the Formula (Ig)

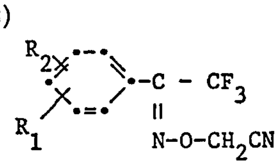

(Ig)

wherein $R_1$ and $R_2$ are as defined in Claim 1.

9. An oxime ether according to Claim 1 of the Formula (Ih)

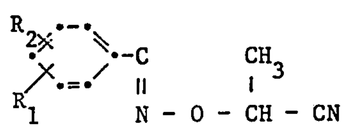

(Ih)

wherein $R_1$ and $R_2$ are as defined in Claim 1.

10. An oxime ether according to Claim 1 of the Formula (Ii)

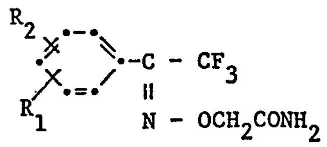

(Ii)

wherein $R_1$ and $R_2$ are as defined in Claim 1.

11. A oxime ether according to Claim 1 of the Formula (Ij)

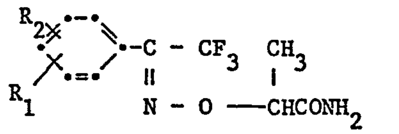 (Ij)

wherein $R_1$ and $R_2$ are as defined in Claim 1.

12. An oxime ether according to Claim 1, wherein $R_1$ is hydrogen, halogen, methyl, methoxy, trifluoromethyl or nitro, $R_2$ is hydrogen, halogen or methyl, and $R_3$ is hydrogen.

13. 1-Phenyl-1-methoximino-2,2,2-trifluoroethane according to Claim 1 of the formula

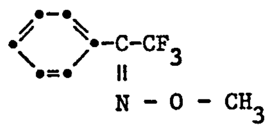

14. 1-(3-Trifluoromethylphenyl)-1-cyclopropylmethoximino-2,2,2-trifluoroethane according to Claim 1 of the formula

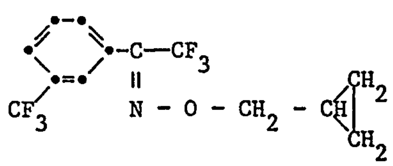

15. 1-(4-Chlorophenyl)-1-n-propoximino-2,2,2-trifluoroethane according to Claim 1 of the formula

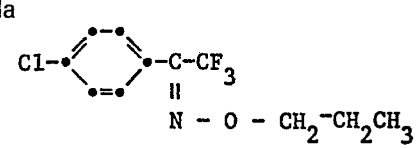

16. 1-(4-Chlorophenyl)-1-allyloximino-2,2,2-trifluoroethane according to Claim 1 of the formula

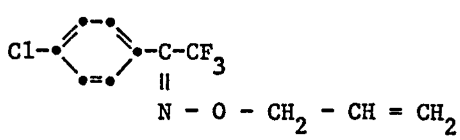

17. 1-Phenyl-1-carbamoylmethoximino-2,2,2-trifluoroethane according to Claim 1 of the formula

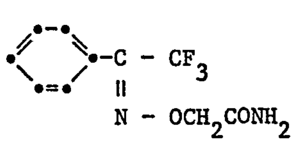

18. 1-(4-Fluorophenyl)-1-carbamoylmethoximino-2,2,2-trifluoroethane according to Claim 1 of the formula

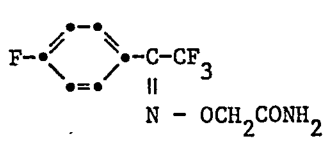

19. 1-(4-Chlorophenyl)-1-carbamoylmethoximino-2,2,2-trifluoroethane according to Claim 1 of the formula

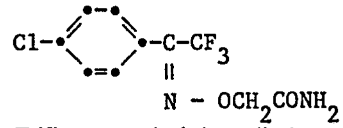

20. 1-(3-Trifluoromethylphenyl)-1-carbamoylmethoximino-2,2,2-trifluoroethane according to Claim 1 of the formula

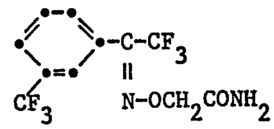

21. 1-Phenyl-1-carbamoylmethoximino-2,2,3,3,3-pentafluoropropane according to Claim 1 of the formula

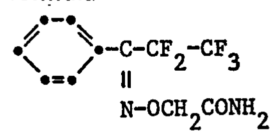

22. 1-Phenyl-1-carbamoyleth-1′-oximino-2,2,2-trifluoroethane according to Claim 1 of the formula

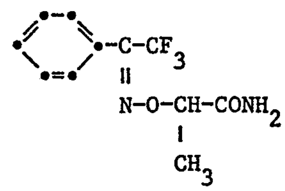

23. 1-(4-Chlorophenyl)-1-carbamoyleth-1′-oximino-2,2,2-trifluoroethane according to Claim 1 of the formula

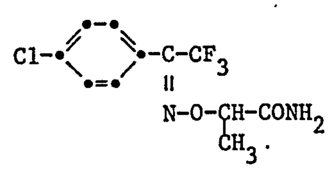

24. 1-(3-Trifluoromethylphenyl)-1-carbamoyleth-1′-oximino-2,2,2-trifluoroethane according to Claim 1 of the formula

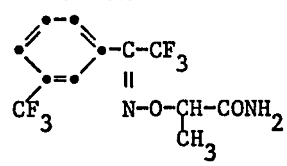

25. 1-(3-Chlorophenyl)-1-isopropoxycarbonylmethoximino-2,2,2-trifluoroethane according to Claim 1 of the formula

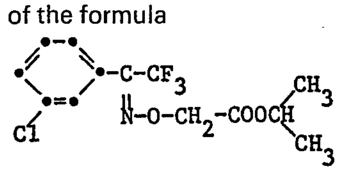

26. A process for the preparation of an oxime ether of the Formula (I) according to Claim 1, which process comprises reacting a salt of an oxime of the Formula (II)

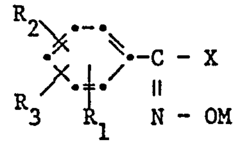 (II)

wherein M is the cation of an alkali metal or alkaline earth metal and $R_1$, $R_2$, $R_3$ and X are as defined in Claim 1, with a reactive ester of the Formula (III)

$$Y-Q \qquad (III)$$

wherein Q is as defined in Claim 1 and Y is the radical of an organic or inorganic acid.

27. A process according to Claim 26, wherein the salt of an oxime of the Formula (II) is the sodium or potassium salt.

28. A process according to Claim 26, wherein Y is a halogen atom or a sulfonic acid radical.

29. A process according to Claim 26, wherein the reaction of the oxime of the Formula (II) with the reactive ester of the Formula (III) is carried out in a polar solvent.

30. A process according to Claim 26, wherein the hydroximine of the Formula (II), or a salt thereof, is reacted with a sulfonyl ester of the Formula (IV)

$$Z-SO_3-Q \qquad (IV)$$

wherein Z is a lower alkyl radical or a phenyl radical which is substituted by lower alkyl or halogen, and Q is as defined in Claim 1.

31. A composition for protecting cultivated plants from damage caused by herbicides, which contains an oxime ether of the Formula (I) according to Claim 1, together with inert carriers and adjuvants.

32. A composition according to Claim 31, which contains

a) a herbicidal haloacetanilide or a herbicidal thiocarbamate, and

b) an oxime ether of the Formula (I) according to Claim 1 as antidote.

33. A composition according to Claim 31 which contains as active component

a) a haloacetanilide herbicide of the Formula (VII)

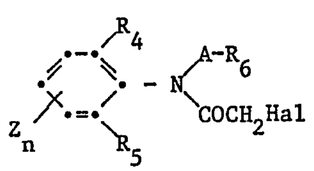 (VII)

wherein Hal is halogen, preferably chlorine or bromine, each of $R_4$ and $R_5$ independently of the other is hydrogen, halogen, lower alkyl, alkoxy, alkylthio, haloalkyl, alkoxyalkyl or alkylthioalkyl, Z is hydrogen, halogen, lower alkyl, alkoxy, alkylthio, haloalkyl, alkoxyalkyl or alkylthioalkyl, which radicals Z are preferably in the 3-position with respect to the nitrogen atom, n is 0 to 3, A is alkylene, preferably methylene, 1,1-ethylene, and 1,2-ethylene which may be substituted by 1 or 2 lower alkyl groups, and $R_6$ is lower alkoxy, hydroxycarbonyl, alkoxycarbonyl, carbamoyl, N-alkylcarbamoyl, N,N-dialkylcarbamoyl, cyano, an unsubstituted or substituted nitrogen-containing heterocyclic radical, alkanoyl, unsubstituted or substituted 1,3,4-oxadiazol-2-yl, 1,3,4-thiadiazol-2-yl, 1,3,4-triazol-3-yl or 1,3,4-triazol-1-yl, and

b) an oxime ether of the Formula (I) according to Claim 1 as antidote.

34. A composition according to Claim 31 which contains

a) a thiocarbamate of the Formula (VIII) or (IX)

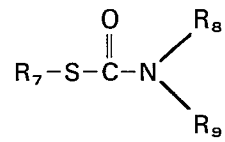 (VIII)

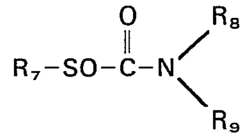 (IX)

wherein $R_7$ is lower alkyl, alkenyl, chloroallyl, dichloroallyl, trichlorallyl, benzyl or 4-chlorobenzyl, $R_8$ is $C_2$-$C_4$-alkyl and $R_9$ is $C_2$-$C_4$-alkyl or cyclohexyl, and $R_8$ and $R_9$ together with the nitrogen atom to which they are attached can form a hexahydro-1H-azepine, decahydroquinoline or 2-methyldecahydroquinoline ring, and

b) an oxime ether of the Formula (I) according to Claim 1 as antidote.

35. Use of an oxime ether of the Formula (I) according to Claim 1 for protecting cultivated plants from damage caused by the application of herbicides.

36. A method of protecting cultivated plants from damage caused by the application of herbicides, which method comprises

a) treating the locus of the plant before or during application of the herbicide, or

b) treating the seeds or seedlings of the plant itself with an effective amount of an oxime ether of the Formula (I) according to Claim 1.

## Revendications

1. Ethers d'oximes de formule (I)

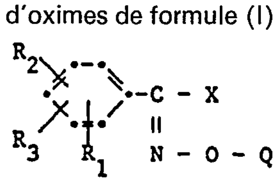 (I)

dans laquelle $R_1$ représente l'hydrogène, un halogène, un groupe alkyle inférieur, halogénoalkyle inférieur, alcoxy inférieur, halogénoalcoxy inférieur, alkylthio inférieur, halogénoalkylthio inférieur, alkylsulfinyle inférieur, alkylsulfonyle inférieur, halogénoalkylsulfinyle inférieur, halogénoalkylsulfonyle inférieur ou nitro,

$R_2$ et $R_3$ représentent chacun l'hydrogène, un halogène, un groupe alkyle inférieur, alcoxy inférieur ou halogéno-alkyle inférieur,

Q représente un groupe alkyle inférieur qui est à chaîne droite ou ramifiée ou est interrompu par des hétéro-atomes, et

a) est substitué par des atomes d'halogène, le groupe cyano ou oxy, ou bien

b) est substitué par un groupe phénoxy ou thiophénoxy ou bien représente

– un groupe alcényle ou halogénoalcényle inférieur,

– un groupe alcynyle ou halogénoalcynyle inférieur,
– un groupe cycloalkyle contenant 3 à 7 chaînons,
– un reste d'ester d'acide alcanecarboxylique inférieur,
– un reste d'ester d'acide alcénylcarboxylique inférieur,
– un reste d'amide ou d'hydrazine d'acide alcanecarboxylique inférieur,
– un reste de thioamide d'acide alcanecarboxylique inférieur,
– un reste d'ester de thiol et d'acide alcanecarboxylique inférieur,
– un reste d'acide alcanecarboxylique inférieur ou un sel d'un tel reste,
– un reste acyle aliphatique ou araliphatique,
– un reste acyle aliphatique halogéno substitué,
– un reste acyle aliphatique portant un substituant alcoxy ou phénoxy,
– un reste acyle cycloaliphatique, aromatique ou hétérocyclique,
– un reste aryle substitué ou non substitué,
– un reste d'acide carbonique aliphatique, cycloaliphatique ou aromatique,
– un reste d'acide thiocarbonique aliphatique, cycloaliphatique ou aromatique,
– le reste carbamoyle, un reste cyanoalkylcarbamoyle ou bien encore, lorsque X ne représente pas le groupe trifluorométhyle, un reste alkylcarbamoyle,
– un reste alkyl- ou arylsulfonyle,
– un reste alcoxyalkylsulfonylamido ou un reste sulfonylamido,
– un reste alkyl- ou arylsulfonyle halogéno substitué,
– un reste acylamidométhyle cyclique

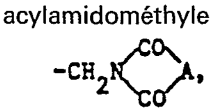

dans lequel

A représente une chaîne alkylène, une chaîne alkylène halogénée, une chaîne alcényle ou une chaîne alcénylène halogénée,
– un reste phtalimidométhyle qui peut être partiellement ou totalement saturé et peut porter sur le noyau phényle des substituants tels que $R_1$, $R_2$ et $R_3$,
– un hétérocycle saturé, partiellement saturé ou aromatique de 5 à 6 chaînons, qui peut contenir 1 à 3 atomes d'azote, d'oxygène et/ou de soufre, et peut porter des substituants tels que $R_1$, $R_2$ et $R_3$, et

X représente un groupe alkyle fluoré en $C_1$-$C_3$ qui peut également contenir du chlore.

2. Ethers d'oximes selon la revendication 1, de formule (Ia)

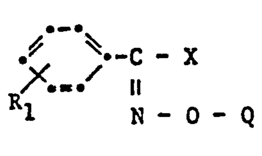 (Ia)

dans laquelle $R_1$, Q et X ont les significations indiquées dans la revendication 1.

3. Ethers d'oximes selon la revendication 1, de formule (Ib)

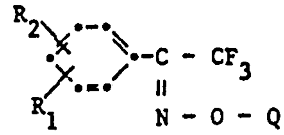 (Ib)

dans laquelle $R_1$ et Q ont les significations indiquées dans la revendication 1.

4. Ethers d'oximes selon la revendication 1 de formule (Ic)

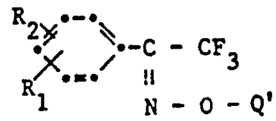 (Ic)

dans laquelle Q' représente un groupe alkyle en $C_1$-$C_4$, cyanalkyle en $C_1$-$C_4$, carbamoyle, $CONH_2$ et $R_1$ et $R_2$ ont les significations indiquées dans la revendication 1.

5. Ethers d'oximes selon la revendication 1, de formule (Id)

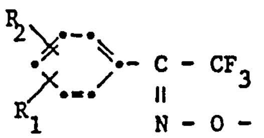 (Id)

dans laquelle Q'' représente un groupe alkyle en $C_1$-$C_4$ ou cyanalkyle en $C_1$-$C_4$ et $R_1$ a la signification indiquée dans la revendication 1.

6. Ethers d'oximes selon la revendication 1, de formule (Ie)

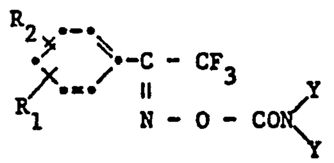 (Ie)

dans laquelle Y représente l'hydrogène et $R_1$ et $R_2$ ont les significations indiquées dans la revendication 1.

7. Ethers d'oximes selon la revendication 1, de formule (If)

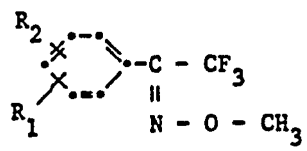 (If)

dans laquelle $R_1$ a la signification indiquée dans la revendication 1.

8. Ethers d'oximes selon la revendication 1 de formule (Ig)

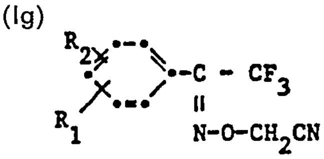 (Ig)

dans laquelle $R_1$ et $R_2$ ont les significations indiquées dans la revendication 1.

9. Ethers d'oximes selon la revendication 1, de formule (Ih)

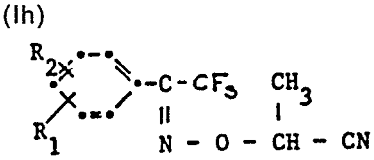 (Ih)

dans laquelle $R_1$ et $R_2$ ont les significations indiquées dans la revendication 1.

10. Ethers d'oximes selon la revendication 1 de formule (Ii)

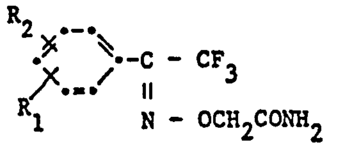

(Ii)

dans laquelle $R_1$ et $R_2$ ont les significations indiquées dans la revendication 1.

11. Ethers d'oximes selon la revendication 1, de formule (Ij)

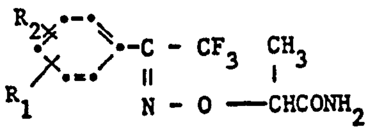

(Ij)

dans laquelle $R_1$ et $R_2$ ont les significations indiquées dans la revendication 1.

12. Ethers d'oximes selon la revendication 1, dans laquelle $R_1$ représente l'hydrogène, un halogène, un groupe méthyle, méthoxy, trifluoro méthyle ou nitro, $R_2$ représente l'hydrogène, un halogène ou un groupe méthyle et $R_3$ l'hydrogène.

13. Le 1-phényl-1-méthoximino-2,2,2-trifluoroéthane de formule (I), de formule

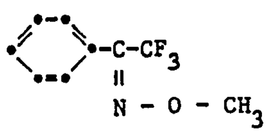

14. Le 1-(3-trifluorométhylphényl)-1-cyclopropylméthoximino-2,2,2-trifluoréthane selon la revendication 1, de formule

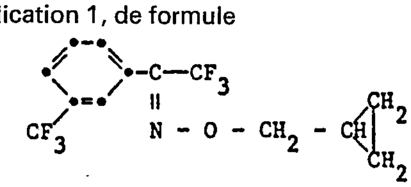

15. Le 1-(4-chlorophényl)-1-n-propoximino-2,2,2-trifluoréthane selon la revendication 1 de formule

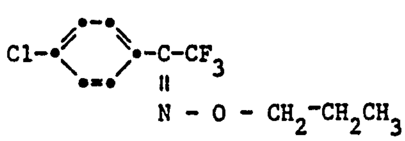

16. Le 1-(4-chlorophényl)-1-allyloximino-2,2,2-trifluoréthane selon la revendication 1 de formule

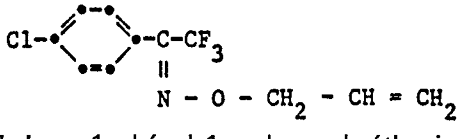

17. Le 1-phényl-1-carbamoylméthoximino-2,2,2-trifluoréthane selon la revendication 1 de formule

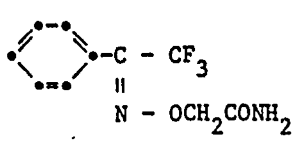

18. Le 1-(4-fluorophényl)-1-carbamoylméthoximino-2,2,2-trifluoréthane selon la revendication 1 de formule

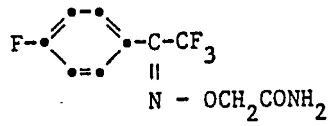

19. Le 1-(4-chlorophényl)-1-carbamoylméthoximino-2,2,2-trifluoréthane selon la revendication 1 de formule

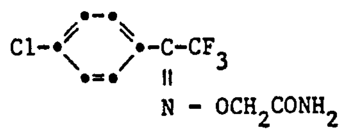

20. Le 1-(3-trifluorométhylphényl)-1-carbamoylméthoximino-2,2,2-trifluoréthane selon la revendication 1 de formule

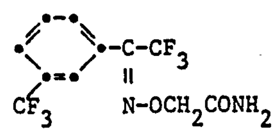

21. Le 1-phényl-1-carbamoylméthoximino-2,2,3,3,3-pentafluoropropane selon la revendication 1, de formule

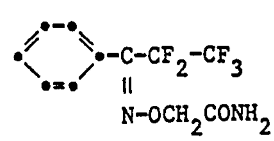

22. Le 1-phényl-1-carbamoyléthyl-1'-oxyimino-2,2,2-trifluoréthane selon la revendication 1, de formule

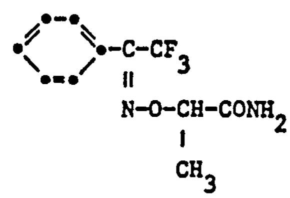

23. Le 1-(4-chlorophényl)-1-carbamoyléthyl-1'-oximino-2,2,2-trifluoréthane selon la revendication 1, de formule

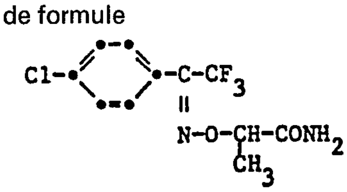

24. Le 1-(3-trifluorométhylphényl)1-carbamoyléthyl-1'-oximino-2,2,2-trifluoréthane selon la revendication 1, de formule

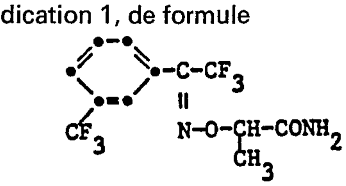

25. Le 1-(3-chlorophényl)-1-isopropyloxycarbonylméthoximino-2,2,2-trifluoréthane selon la revendication 1, de formule

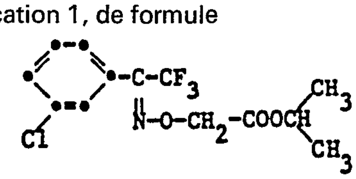

26. Procédé de préparation des éthers d'oximes de formule (I), selon la revendication 1, caractérisé

en ce que l'on fait réagir un sel d'une oxime de formule (II)

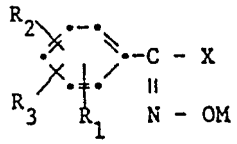

(II)

dans laquelle M représente un cation de métal alcalin ou alcalino-terreux et $R_1$, $R_2$, $R_3$ et X ont les significations indiquées dans la revendication 1, avec un ester réactif de formule (III)

$$Y-Q \qquad (III)$$

dans laquelle Q a les significations indiquées dans la revendication 1, et Y représente un reste d'acide organique ou minéral.

27. Procédé selon la revendication 17, caractérisé en ce que l'on utilise en tant que sel d'une oxime de formule (II) le sel de sodium ou de potassium.

28. Procédé selon la revendication 17, caractérisé en ce que B représente un atome d'halogène ou un reste d'acide sulfonique.

29. Procédé selon la revendication 17, caractérisé en ce que la réaction entre l'oxime de formule (II) et l'ester réactif de formule (III) est effectuée dans un solvant polaire.

30. Procédé selon la revendication 17, caractérisé en ce que l'on fait réagir l'hydroximine de formule (II) ou un sel de ce composé avec un ester sulfonylé de formule (IV)

$$Z-SO_3-Q \qquad (IV)$$

dans laquelle Z représente un groupe alkyle inférieur ou un groupe phényle portant des substituants alkyle inférieurs ou halogéno et Q a les significations indiquées dans la revendication 1.

31. Produit pour protéger les végétaux cultivés contre les dommages provoqués par les herbicides, caractérisé en ce qu'il contient en tant que composant actif, avec des véhicules et additifs inertes, un éther d'oxime de formule (I) selon la revendication 1.

32. Produit selon la revendication 31, caractérisé en ce qu'il contient:

a) un halogénoacétanilide herbicide actif ou un thiolcarbamate herbicide actif, et

b) un éther d'oxime de formule (I) selon la revendication 1 en tant qu'antidote.

33. Produit selon la revendication 32, caractérisé en ce qu'il contient:

a) un halogénoacétanilide de formule (VII)

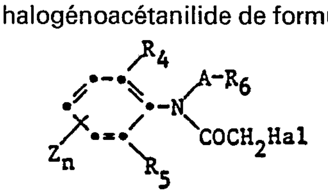

(VII)

dans laquelle Hal représente un halogène, en particulier le fluor ou le brome, $R_4$ et $R_5$ représentent chacun, indépendamment l'un de l'autre, l'hydrogène, un halogène, un groupe alkyle inférieur, alcoxy, alkylthio, halogénoalkyle, alcoxyalkyle ou alkylthioalkyle, Z représente l'hydrogène, un halogène, un groupe alkyle inférieur, alcoxy, alkylthio, halogénoalkyle, alcoxyalkyle ou alkylthioalkyle, les restes Z sont de préférence en position 3 par rapport à l'atome d'azote, n a une valeur de 0 à 3, A représente un groupe alkylène, en particulier méthylène, 1,1- et 1,2-éthylène, un groupe 1,2-éthylène pouvant être substitué par 1 ou 2 groupes alkyle inférieurs, et $R_6$ représente un groupe alcoxy inférieur, hydroxycarbonyle, alcoxycarbonyle, carbamoyle, N-alkylcarbamoyle, N,N-dialkylcarbamoyle, cyano, un reste hétérocyclique azoté éventuellement susbtitué, un groupe alcanoyle, un groupe benzyle éventuellement substitué, un groupe 1,3,4-oxadiazole-2-yle, 1,3,4-thiadiazole-2-yle, 1,3,4-triazole-3-yle ou 1,3,4-triazole-1-yle éventuellement substitué, et

b) un éther d'oxime de formule (I) selon la revendication 1 en tant qu'antidote.

34. Produit selon la revendication 32, caractérisé en ce qu'il contient:

a) un thiolcarbamate de formules (VIII) et (IX)

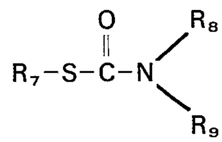

(VIII)

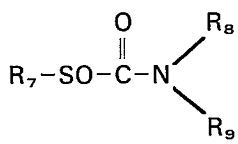

(IX)

dans laquelle $R_7$ représente un groupe alkyle inférieur, alcényle, chlorallyle, dichlorallyle, trichlorallyle, benzyle ou 4-chlorobenzyle, $R_8$ représente un groupe alkyle en $C_2$-$C_4$ et $R_9$ représente un groupe alkyle en $C_2$-$C_4$ ou cyclohexyle, les restes $R_8$ et $R_9$ pouvant former avec l'atome d'azote auquel ils sont reliés un cycle hexahydro-1H-azépine, décahydroquinoléine ou 2-méthyldécahydroquinoléine, et

b) un éther d'oxime de formule (I) selon la revendication 1 en tant qu'antidote.

35. Utilisation des éthers d'oximes de formule (I) selon la revendication 1 pour la protection des végétaux cultivés contre les effets nocifs des herbicides.

36. Procédé pour protéger les végétaux cultivés contre les dommages provoqués par l'application des herbicides, caractérisé en ce que

a) on traite l'aire de culture de la plante, avant ou après l'application de l'herbicide, ou bien

b) on traite les graines ou les plants de la plante ou la plante elle-même par une quantité efficace d'un éther d'oxime de formule (I).